# FASCICULE DE BREVET EUROPEEN

(11) **EP 0 763 602 B1**
(45) Date de publication et mention de la délivrance du brevet: **10.09.2003**
(21) Numéro de dépôt: 96420293.1
(22) Date de dépôt: 17.09.1996
(51) Int. Cl.: C12Q 1/68

(54) **Détection des Entérobactéries**
Nachweis von Enterobakterien
Detection of enterobacteria

(30) Priorité: 18.09.1995 FR 9511125
(43) Date de publication de la demande: 19.03.1997
(73) Titulaire: BIO MERIEUX, 69280 Marcy l'Etoile (FR); Université de la Mériterranée, AIX-Marseille II, 13284 Marseille Cedex 07 (FR)
(72) Inventeur: Mabilat, Claude, 69140 Rillieux la Pape (FR); Raoult, Didier, 13008 Marseille (FR)
(74) Mandataire: Guerre, Dominique

(56) Documents cités:
- WO-A-93/22454
- MANFREDI ET AL.: "Rearrangements in unitegrated retroviral DNA are complex and are the result of multiple genetic determinants" JOURNAL OF VIROLOGY, vol. 64, 1990, pages 5475-5484, XP000574757
- LAZCANO ET AL.: "On the early evolution of RNA polymerase" JOURNAL OF MOLECULAR EVOLUTION, vol. 27, 1988, pages 365-376, XP000574039 NEW YORK, US
- POST ET AL.: "Nucleotide sequence of the ribosomal protein gene cluster adjacent to the gene for RNA polymerase subunit beta in Escherichia coli" PROC. NATL. ACAD. SCI. U.S.A. 76:1697-1701(1979), XP002007059
- LYSITSYN ET AL.: "Genes coding for RNA polymerase beta subunit in bacteria. Structure/function analysis." EUROPEAN JOURNAL OF BIOCHEMISTRY, (1988 NOV 1) 177 (2) 363-9., XP002113809
- BUSTAMANTE ET AL.: "Identification of Campylobacter jejuni and C. coli using the rpoB gene and a cryptic DNA fragment from C. jejuni" GENE, (1995 NOV 7) 165 (1) 1-8., XP004043174

## Description

La présente invention relève du domaine des techniques de détection et/ou d'amplification, à l'aide de sondes ou d'amorces oligonucléotidiques, et leur application à la recherche de la présence ou à l'identification des bactéries du genre Enterobacteriaceae.

Dans le cadre de certains tests de diagnostic, notamment lors de recherche d'infection septicémique ou dans le domaine alimentaire, il est souvent nécessaire d'obtenir une réponse rapide concernant la présence de bactéries, en particulier de bactéries gram négatif, et plus précisément d'entérobactéries, dans un échantillon. Toutefois, les techniques couramment développées (coloration de Gram, identification biochimique,...) nécessitent une étape préalable de culture, souvent dans des conditions très spécifiques (hémoculture, par exemple), en raison de la faible proportion en particules bactériennes présentes dans l'échantillon de départ.

Une solution possible réside dans l'utilisation des technologies relatives aux acides nucléiques et au matériel génétique, afin de déterminer si un gène, une partie de gène ou une séquence nucléotidique est présent chez un organisme vivant, un extrait cellulaire de cet organisme ou un échantillon. Etant donné que tout gène ou partie de gène est caractérisé par une séquence spécifique de bases nucléotidiques, il est par conséquent possible de rechercher directement la présence de tout ou partie de ladite séquence spécifique au sein d'un échantillon contenant un mélange de polynucléotides.

Différents types de méthodes de détection des acides nucléiques sont décrits dans la littérature. Ces méthodes reposent sur les propriétés d'appariement purine-pyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénine-thymine (A-T) et guanine-cytosine (G-C) de l'ADN double brin ; des paires de bases adénine-uracile (A-U) peuvent également se former par liaison hydrogène dans les duplex ADN-ARN ou ARN-ARN. L'appariement de brins d'acide nucléique pour la détermination de la présence ou de l'absence d'une molécule d'acide nucléique donnée est communément appelée "hybridation d'acides nucléiques" ou simplement "hybridation".

Selon la présente invention, on a identifié de nouveaux marqueurs génétiques permettant la détection spécifique de bactéries appartenant à la famille des Entérobactéries dans tout échantillon, ne nécessitant aucune étape préalable de culture bactérienne. L'invention repose sur l'utilisation de séquences nucléiques spécifiquement définies dans le gène rpoB codant pour une des sous-unités de l'ARN polymérase bactérienne.

Selon Lazcano et al., 1988, les ARN polymérases sont divisées en deux groupes selon leur origine, l'un constitué par les ARN polymérases virales ARN ou ADN dépendantes, et l'autre constitué par les ARN polymérases ADN-dépendantes d'origine eucaryote ou procaryote (archaebactéries et eubactéries). Les ARN polymérases ADN-dépendantes eubactériennes sont caractérisées par une constitution multimérique simple et conservée notée "core enzyme" et figurée par αββ' ou "holoenzyme" et figurée par αββ'α' [Burgess et al., J. Biol. Chem. (1969) 244 : 6168-6176; Chamberlin, Ann. Rev. Biochem. (1974) 43 : 721-775 ; Yura and Ishihama, Ann. Rev. Genet. (1979) 13 : 59-97 ; Sentenac, CRC - Crit. Rev. Biochem. (1985) 18(1) : 31-90]. De nombreux travaux ont mis en évidence le rôle fonctionnel, au sein du complexe enzymatique multimérique, de la sous-unité β de l'ARN polymérase eubactérienne. Les ARN polymérases archaebactérienne et eucaryote présentent, pour leur part, une structure plus complexe pouvant atteindre une dizaine, voire une trentaine, de sous-unités [Pühler et al. - Proc. Natl. Acad. Sci. USA (1989) 86 : 4569-4573; Sentenac, CRC - Crit. Rev. Biochem. (1985) 18(1) : 31-90].

Les gènes qui codent pour les différentes sous-unités de l'ARN polymérase ADN-dépendante chez les eubactéries, respectivement rpoA, rpoB, rpoC et rpoD pour αββ'α', sont regroupés en différents segments comprenant des gènes codant pour des protéines constitutives des sous-unités ribosomiques ou pour des enzymes impliquées dans la réplication et la réparation du génome [Yura and Yshihama, Ann. Rev. Genet. (1979) 13 : 59-97]. Certains auteurs ont montré que les séquences nucléiques des gènes rpoB et rpoC pouvaient être utilisées afin de construire des arbres phylogénétiques [Lazcano et al., J. Mol. Evol. (1988) 27 : 365-376 ; Zillig et al., Can. J. Microbiol. (1989) 35 : 73-80 ; Rowland et al., Biochem. Soc. Trans. (1992) 21 : 40S] permettant de séparer les différents embranchements et sous-embranchements parmi les règnes du vivant. En outre, il a été montré, chez les eubactéries, que la sous-unité β constitue la molécule cible des rifamycines (dont la rifampicine), des streptovaricines et de la streptolydigine [Kumar and Chatterji, Biochemistry (1990) 29 : 317-322 ; Kumar et al., Biochemistry (1992) 31 : 7519-7526] et que le gène rpoB constitue le support génétique de la résistance des germes à ces antibiotiques [Ovchinnikov et al., Mol. Gen. Genet. (1983) 190 : 344-348 ; Lisitsyn et al., Mol. Gen. Genet. (1984) 196 : 173-174]. De nombreuses mutations du gène rpoB affectant la régulation de l'expression et de la fonction de l'ARN polymérase ont ainsi été décrites [Landick et al, Genes Dev. (1990) 4 : 1623-1636] dont certaines sont liées à des mutations conférant le phénotype résistant à la rifampicine [Jin and Gross, J. Biol. Chem. (1991) 266 : 14478-14485 ; Jin and Turnbough, J. Mol. Biol. (1994) 236 : 72-80]. La détermination de la résistance à la rifampicine de souches *Mycobacterium tuberculosis* par l'utilisation du gène rpoB a fait l'objet de plusieurs travaux (Hunt et al., 1994, Diagn. Microbiol. Infect. Dis., 18, 219-227 ; Whelen et al., 1995, J. Cli. Microbiol. 33, 556-561 ; et demande de brevet n° WO 9322454).

On a maintenant découvert sur l'ADN codant pour la sous unité β de l'ARN polymérase bactérienne des zones variables selon les familles bactériennes, mais qui apparaissent conservées parmi la famille des *Enterobacteriaceae*, ce qui permet de discriminer cette famille bactérienne parmi d'autres familles bactériennes. Par ailleurs, il existe dans lesdites zones conservées, des variations mineures de séquence entre certaines espèces entérobactériennes. Ces résultats ont permis de concevoir des sondes nucléiques spécifiques soit de la famille entérobactérienne, soit de certaines espèces particulières.

L'art antérieur est en outre constitué par les documents suivants WO-A-94/24565, EP-0 473 268 et KANELLOPOULOS, et al., Journal of Experimental Medicine, vol. 180, 1994, p. 861-872. Comme mentionné ci-après, ces documents décrivent des séquences nucléotidiques possédant une suite de motifs nucléotidiques identique à certaines des séquences rapportées par la présente invention, mais ni ne révèlent, ni ne suggèrent leur utilisation pour détecter des entérobactéries.

Ainsi le document WO-A-94/24565 concerne la détection d'antigènes du VHC dans un échantillon à l'aide d'anticorps spécifiques des antigènes du VHC et décrit la séquence identifiée dans ce document par SEQ ID NO 11 décrite à la page 71. Cette séquence est identifiée dans la présente description par SEQ ID NO 77.

Le document EP-0 473 268 enseigne une composition pharmaceutique comprenant un principe actif polypeptidique, et permettant une libération progressive du polypeptide actif. Il décrit la séquence identifiée dans ce document par SEQ ID NO 19, et dans la présente description par SEQ ID NO 78.

L'article de KANELLOPOULOS et al., concerne une souris transgénique et décrit, à la ligne 17 de la page 866, la séquence que l'on a identifié dans la présente description par SEQ ID NO 79.

Avant d'exposer plus en détail l'invention, différents termes utilisés dans la description et les revendications sont définis ci-après :
- par "acide nucléique extrait de bactéries" on entend soit l'acide nucléique total, soit l'ADN génomique, soit les ARN messagers, soit encore l'ADN obtenu à partir de la transcription inverse des ARN messagers ;
- un "fragment nucléotidique" ou un "oligonucléotide" sont deux termes synonymes désignant un enchaînement de motifs nucléotidiques caractérisé par la séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminées. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 10, et en particulier au moins 12 motifs nucléotidiques et peut être obtenu à partir d'une molécule d'acide nucléique naturelle et/ou par recombinaison génétique et/ou par synthèse chimique,
- un motif nucléotidique est dérivé d'un monomère qui peut être un nucléotide naturel d'acide nucléique dont les éléments constitutifs sont un sucre, un groupement phosphate et une base azotée ; dans l'ADN le sucre est le désoxy-2-ribose, dans l'ARN le sucre est le ribose ; selon qu'il s'agit d'ADN ou d'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl-5-désoxycytidine, la désoxyuridine, la diméthylamino-5-désoxyuridine, la diamino-2,6-purine, la bromo-5-désoxyuridine ou toute autre base modifiée capable d'hybridation, soit au niveau du sucre, par exemple le remplacement d'au moins un désoxyribose par un polyamide [P.E. Nielsen et al., Science, 254, 1497-1500 (1991)], soit encore au niveau du groupement phosphate, par exemple son remplacement par des esters notamment choisis parmi les diphosphates, alkyl- et aryl-phosphonates et phosphorothioates,
- par "séquence informationnelle", on entend toute suite ordonnée de motifs de type nucléotidique, dont la nature chimique et l'ordre dans un sens de référence constituent une information analogue à celle donnée par la séquence des acides nucléiques naturels,
- par "hybridation", on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la "stringence", c'est-à-dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65°C, en particulier entre 35 et 65°C dans une solution saline à une concentration d'environ 0,8 à 1M.
- une "sonde" est un fragment nucléotidique comprenant par exemple de 10 à 100 motifs nucléotidiques, notamment de 12 à 35 motifs nucléotidiques, possédant une spécificité d'hybridation dans des conditions déterminées pour former un complexe d'hybridation avec un acide nucléique cible ayant, dans le cas présent, une séquence nucléotidique comprise soit dans un ARN messager, soit dans un ADN obtenu par transcription inverse dudit ARN messager, soit encore dans un ADN dont ledit ARN messager est le produit de transcription; une sonde peut être utilisée à des fins de diagnostic (notamment sondes de capture ou de détection) ou à des fins de thérapie,
- une "sonde de capture" est immobilisée ou immobilisable sur un support solide par tout moyen approprié, par exemple par covalence, par adsorption, ou par synthèse directe sur un support solide (voir notamment la demande de brevet WO 92 10092)
- une "sonde de détection" peut être marquée au moyen d'un marqueur choisi par exemple parmi les isotopes radioactifs, des enzymes, en particulier des enzymes susceptibles d'agir sur un substrat chromogène, fluorigène ou luminescent (notamment une peroxydase ou une phosphatase alcaline), des composés chimiques chromophores, des composés chromogènes, fluorigènes ou luminescents, des analogues de bases nucléotidiques, et des ligands tels que la biotine,
- une "amorce" est une sonde comprenant par exemple de 10 à 100 motifs nucléotidiques et possédant une spécificité d'hybridation dans des conditions déterminées pour l'initiation d'une polymérisation enzymatique, par exemple dans une technique d'amplification telle que la PCR (Polymerase Chain Reaction), dans un procédé de séquençage, dans une méthode de transcription inverse, etc.,

Un premier objet de la présente invention est un oligonucléotide monocaténaire choisi parmi les oligonucléotides ayant une séquence d'au moins 12 motifs nucléotidiques consécutifs incluse dans l'une des séquences SEQ ID NO1 à SEQ ID NO52, et parmi les oligonucléotides complémentaires de ces oligonucléotides, à l'exclusion des oligonucléotides ayant une séquence choisie parmi les séquences suivantes : SEQ ID NO 28, SEQ ID NO 77, SEQ ID NO 78 et SEQ ID NO 79.

Des oligonucléotides préférés de l'invention sont a) ceux qui comprennent une séquence nucléotidique d'au moins 12 motifs nucléotidiques consécutifs incluse dans l'une des séquences SEQ ID NO1 à SEQ ID NO4, et leurs séquences complémentaires, et b) ceux qui comprennent une séquence nucléotidique d'au moins 12 motifs nucléotidiques consécutifs incluse dans :
SEQ ID NO5 et contenant au moins 5 motifs nucléotidiques consécutifs inclus dans SEQ ID NO53,
SEQ ID NO6 et contenant au moins 5 motifs nucléotidiques consécutifs inclus dans SEQ ID NO54,
SEQ ID NO8 et contenant au moins 5 motifs nucléotidiques consécutifs inclus dans SEQ ID NO56,
SEQ ID NO8 et contenant au moins 5 motifs nucléotidiques consécutifs inclus dans SEQ ID NO57,
SEQ ID NO9 et contenant au moins 5 motifs nucléotidiques consécutifs inclus dans SEQ ID NO58,
SEQ ID NO10 et contenant au moins 5 motifs nucléotidiques consécutifs inclus dans SEQ ID NO59,
SEQ ID NO12 et contenant au moins 5 motifs nucléotidiques consécutifs inclus dans SEQ ID NO61,
SEQ ID NO13 et contenant au moins 5 motifs nucléotidiques consécutifs inclus dans SEQ ID NO62,
SEQ ID NO14 et contenant au moins 5 motifs nucléotidiques consécutifs inclus dans SEQ ID NO63,
SEQ ID NO16 et contenant au moins 5 motifs nucléotidiques consécutifs inclus dans SEQ ID NO65,
SEQ ID NO17 et contenant au moins 5 motifs nucléotidiques consécutifs inclus dans SEQ ID NO64,
SEQ ID NO18 et contenant au moins 5 motifs nucléotidiques consécutifs inclus dans SEQ ID NO66,
SEQ ID NO19 et contenant au moins 5 motifs nucléotidiques consécutifs inclus dans SEQ ID NO67,
SEQ ID NO20 et contenant au moins 5 motifs nucléotidiques consécutifs inclus dans SEQ ID NO68,
SEQ ID NO21 et contenant au moins 5 motifs nucléotidiques consécutifs inclus dans SEQ ID NO69,
SEQ ID NO22 et contenant au moins 5 motifs nucléotidiques consécutifs inclus dans SEQ ID NO70,
SEQ ID NO23 et contenant au moins 5 motifs nucléotidiques consécutifs inclus dans SEQ ID NO71,
SEQ ID NO24 et contenant au moins 5 motifs nucléotidiques consécutifs inclus dans SEQ ID NO72,
SEQ ID NO25 et contenant au moins 5 motifs nucléotidiques consécutifs inclus dans SEQ ID NO73,
SEQ ID NO26 et contenant au moins 5 motifs nucléotidiques consécutifs inclus dans SEQ ID NO74,
SEQ ID NO27 et contenant au moins 5 motifs nucléotidiques consécutifs inclus dans SEQ ID NO75,
ou qui comprennent une séquence complémentaire.

Des oligonucléotides b) préférés comprennent ou consistent en une séquence nucléotidique choisie parmi les séquences SEQ ID NO29 à SEQ ID NO52.

Un second objet de l'invention est une sonde pour la détection, dans un échantillon biologique, de bactéries appartenant à la famille des entérobactéries, ladite sonde consistant en un oligonucléotide de l'invention selon a). Dans la suite de la description, une telle sonde de l'invention sera appelée sonde de genre.

Un troisième objet de l'invention est une sonde pour la détection, dans un échantillon biologique, d'au moins une espèce d'entérobactéries, ladite sonde consistant en un oligonucléotide de l'invention selon b). De telles sondes de l'invention seront appelées sondes d'espèce, dans la présente description.

Les sondes selon l'invention peuvent être utilisées, à des fins de diagnostic, dans la recherche de la présence ou de l'absence d'un acide nucléique cible dans un échantillon, selon toutes les techniques d'hybridation connues et notamment les techniques de dépôt ponctuel sur filtre, dites "DOT-BLOT" (MANIATIS et al, Molecular Cloning, Cold Spring Harbor, 1982), les techniques de transfert d'ADN dites "SOUTHERN BLOT" [SOUTHERN. E.M., J. Mol. Biol., 98, 503 (1975)], les techniques de transfert d'ARN dites "NORTHERN BLOT", ou les techniques dites "sandwich" [DUNN A.R., HASSEL J.A., Cell, 12, 23 (1977)] ; on utilise en particulier la technique sandwich, avec une sonde de capture et/ou une sonde de détection, lesdites sondes étant capables de s'hybrider avec deux régions différentes de l'acide nucléique cible, et l'une au moins desdites sondes (généralement la sonde de détection) étant capable de s'hybrider avec une région de la cible qui est spécifique de l'espèce ou du groupe d'espèces recherché, étant entendu que la sonde de capture et la sonde de détection doivent avoir des séquences nucléotidiques au moins partiellement différentes.

Pour mettre en oeuvre les techniques d'hybridation précitées, et en particulier les techniques "sandwich", une sonde de l'invention est immobilisée sur un support solide, elle sera la sonde de capture, et une autre sonde de l'invention est marquée avec un agent traceur, elle sera la sonde de détection.

L'invention concerne en outre les utilisations d'un oligonucléotide choisi parmi les oligonucléotides ayant une séquence d'au moins 12 motifs nucléotidiques consécutifs incluse dans l'une des séquences SEQ ID NO 1 à SEQ ID NO 52, et parmi les oligonucléotides complémentaires de ces oligonucléotides, comme sonde et comme amorce.

Un autre objet de l'invention est un procédé de détermination de la présence ou de l'absence d'au moins une entérobactérie, dans un échantillon contenant ou susceptible de contenir des acides nucléiques d'au moins une telle bactérie, comprenant les étapes consistant à mettre en contact ledit échantillon avec au moins un oligonucléotide de l'invention tel qu'utilisé comme sonde de genre ou d'espèce, puis à déterminer de façon connue en soi la formation ou l'absence de formation d'un complexe d'hybridation entre ledit oligonucléotide et l'acide nucléique de l'échantillon.

Selon une mise en oeuvre particulière de ce procédé pour la détermination de la présence ou l'absence d'une espèce ou d'un groupe d'espèces d'entérobactérie, on utilise d'une part un oligonucléotide tel qu'utilisé comme sonde de genre selon l'invention et d'autre part un oligonucléotide tel qu'utilisé comme sonde d'espèce selon l'invention, étant entendu que lesdits oligonucléotides sont capables de s'hybrider avec des régions non chevauchantes d'un acide nucléique correspondant au gène rpoB des entérobactéries.

De manière avantageuse, la sonde de genre est immobilisée sur un support solide, et la sonde d'espèce est marquée avec un agent traceur.

La présente invention a aussi pour objet l'application du procédé de l'invention pour déterminer la présence d'une espèce d'entérobactérie déterminée.

Ainsi l'invention concerne :
- un procédé pour la détermination de la présence ou de l'absence de *Salmonella sofia*, selon lequel on utilise une sonde choisie parmi celles comprenant une séquence nucléotidique d'au moins 12 motifs nucléotidiques consécutifs incluse dans l'une des séquences choisies parmi :
   SEQ ID NO5 et contenant au moins 5 motifs nucléotidiques consécutifs inclus dans SEQ ID NO53,
   SEQ ID NO27 et contenant au moins 5 motifs nucléotidiques consécutifs inclus dans SEQ ID NO75, et
leurs séquences complémentaires.

Préférentiellement, une sonde de l'espèce *Salmonella sofia* a une séquence nucléotidique qui consiste en ou qui comprend une séquence choisie parmi SEQ ID NO29, SEQ ID NO51 et, leurs séquences complémentaires ;
- un procédé pour la détermination de la présence ou de l'absence de *Salmonella typhimurium*, selon lequel on utilise une sonde choisie parmi celles comprenant une séquence nucléotidique d'au moins 12 motifs nucléotidiques consécutifs incluse dans l'une des séquences choisies parmi :
   SEQ ID NO6 et contenant au moins 5 motifs nucléotidiques consécutifs inclus dans SEQ ID NO54,
   SEQ ID NO11 et contenant au moins 5 motifs nucléotidiques consécutifs inclus dans SEQ ID NO60,
   SEQ ID NO24 et contenant au moins 5 motifs nucléotidiques consécutifs inclus dans SEQ ID NO72,
   SEQ ID NO25 et contenant au moins 5 motifs nucléotidiques consécutifs inclus dans SEQ ID NO73, et
leurs séquences complémentaires ;

Préférentiellement, une sonde de l'espèce *Salmonella typhimurium* a une séquence nucléotidique qui consiste ou qui comprend une séquence choisie parmi SEQ ID NO30, SEQ ID NO35, SEQ ID NO48, SEQ ID NO49, SEQ ID NO52, et leurs séquences complémentaires ;
- un procédé pour la détermination de la présence ou de l'absence de *Shigella dysenteriae* selon lequel on utilise une sonde choisie parmi celles comprenant une séquence nucléotidique d'au moins 12 motifs nucléotidiques consécutifs incluse dans SEQ ID NO7 et contenant au moins 5 motifs nucléotidiques consécutifs inclus dans SEQ ID NO55 et sa séquence complémentaire ;

Préférentiellement, une sonde de l'espèce *Shigella dysenteriae* a une séquence nucléotidique qui consiste ou qui comprend une séquence choisie parmi SEQ ID NO31, et sa séquence complémentaire ;
- un procédé pour la détermination de la présence ou de l'absence de *Escherichia fergussoni*, selon lequel on utilise une sonde choisie parmi celles comprenant une séquence nucléotidique d'au moins 12 motifs nucléotidiques consécutifs incluse dans l'une des séquences choisies parmi :
   SEQ ID NO8 et contenant au moins 5 motifs nucléotidiques consécutifs inclus dans SEQ ID NO56,
   SEQ ID NO8 et contenant au moins 5 motifs nucléotidiques consécutifs inclus dans SEQ ID NO57,
   SEQ ID NO19 et contenant au moins 5 motifs nucléotidiques consécutifs inclus dans SEQ ID NO67, et
leurs séquences complémentaires ;

Préférentiellement, une sonde de l'espèce *Escherichia fergussoni* a une séquence nucléotidique qui consiste ou qui comprend une séquence choisie parmi SEQ ID NO32, SEQ ID NO43, et leurs séquences complémentaires ;
- un procédé pour la détermination de la présence ou de l'absence de *Enterobacter cloacae,* selon lequel on utilise une sonde choisie parmi celles comprenant une séquence nucléotidique d'au moins 12 motifs nucléotidiques consécutifs incluse dans l'une des séquences choisies parmi :
   SEQ ID NO9 et contenant au moins 5 motifs nucléotidiques consécutifs inclus dans SEQ ID NO58,
   SEQ ID NO13 et contenant au moins 5 motifs nucléotidiques consécutifs inclus dans SEQ ID NO62,
   SEQ ID NO16 et contenant au moins 5 motifs nucléotidiques consécutifs inclus dans SEQ ID NO65,
   SEQ ID NO17 et contenant au moins 5 motifs nucléotidiques consécutifs inclus dans SEQ ID NO64,
   SEQ ID NO18 et contenant au moins 5 motifs nucléotidiques consécutifs inclus dans SEQ ID NO66,
   SEQ ID NO21 et contenant au moins 5 motifs nucléotidiques consécutifs inclus dans SEQ ID NO69,
   SEQ ID NO22 et contenant au moins 5 motifs nucléotidiques consécutifs inclus dans SEQ ID NO70, et
leurs séquences complémentaires ;

Préférentiellement, une sonde de l'espèce *Enterobacter cloacae* a une séquence nucléotidique qui consiste ou qui comprend une séquence choisie parmi SEQ ID NO33, SEQ ID NO37, SEQ ID NO40, SEQ ID NO41, SEQ ID NO42, SEQ ID NO45, SEQ ID NO46, et leurs séquences complémentaires ;
- un procédé pour la détermination de la présence ou de l'absence de *Klebsiella pneumoniae*, selon lequel on utilise une sonde choisie parmi celles comprenant une séquence nucléotidique d'au moins 12 motifs nucléotidiques consécutifs incluse dans l'une des séquences choisies parmi :
   SEQ ID NO10 et contenant au moins 5 motifs nucléotidiques consécutifs inclus dans SEQ ID NO59,
   SEQ ID NO14 et contenant au moins 5 motifs nucléotidiques consécutifs inclus dans SEQ ID NO63,
   SEQ ID NO15 et contenant au moins 5 motifs nucléotidiques consécutifs inclus dans SEQ ID NO64,
   SEQ ID NO20 et contenant au moins 5 motifs nucléotidiques consécutifs inclus dans SEQ ID NO68,
   SEQ ID NO23 et contenant au moins 5 motifs nucléotidiques consécutifs inclus dans SEQ ID NO71, et
leurs séquences complémentaires ;

Préférentiellement, une sonde de l'espèce *Klebsiella pneumoniae* a une séquence nucléotidique qui consiste ou qui comprend une séquence choisie parmi SEQ ID NO34, SEQ ID NO38, SEQ ID NO39, SEQ ID NO44, SEQ ID NO47, et leurs séquences complémentaires.
- un procédé pour la détermination de la présence ou de l'absence de *Escherichia coli*, selon lequel on utilise une sonde choisie parmi celles comprenant une séquence nucléotidique d'au moins 12 motifs nucléotidiques consécutifs incluse dans l'une des séquences choisies parmi :
   SEQ ID NO12 et contenant au moins 5 motifs nucléotidiques consécutifs inclus dans SEQ ID NO61,
   SEQ ID NO26 et contenant au moins 5 motifs nucléotidiques consécutifs inclus dans SEQ ID NO74, et
leurs séquences complémentaires.

Préférentiellement, une sonde de l'espèce *Escherichia coli* a une séquence nucléotidique qui consiste ou qui comprend une séquence choisie parmi SEQ ID NO36, SEQ ID NO50, et leurs séquences complémentaires.

Selon une mise en oeuvre particulière de ces procédés, on utilise les sondes sélectionnées en combinaison avec une sonde de genre de l'invention.

Encore un objet de l'invention est une amorce nucléotidique utilisable pour la synthèse d'un acide nucléique en présence d'une polymérase de façon connue en soi, et notamment dans des méthodes d'amplification utilisant une telle synthèse en présence d'une polymérase (PCR, RT-PCR, etc...), ladite amorce comprenant un oligonucléotide tel que défini précédemment. Notamment, une amorce de l'invention peut être utilisée pour la transcription inverse spécifique d'une séquence d'ARN messager d'au moins une espèce ou d'au moins un groupe d'espèces d'entérobactéries pour obtenir une séquence d'ADN complémentaire correspondante. Une telle transcription inverse peut constituer le premier stade d'une RT-PCR, le stade suivant étant l'amplification par PCR de l'ADN complémentaire obtenu. On peut également utiliser les amorces de l'invention pour l'amplification spécifique par réaction de polymérisation en chaîne de la séquence d'ADN du gène rpoB d'au moins une espèce ou d'au moins un groupe d'espèces d'entérobactérie.

Selon un cas particulier ladite amorce comprenant un oligonucléotide de l'invention comprend en outre la séquence sens ou anti-sens d'un promoteur reconnu par une ARN polymérase (T7, T3, SP6 par exemple) : de telles amorces sont utilisables dans des procédés d'amplification d'acide nucléique faisant intervenir une étape de transcription, tels que, par exemple, les techniques NASBA ou 3SR.

Enfin, un dernier objet de l'invention est une sonde de thérapie pour traiter les infections provoquées par au moins une espèce ou un groupe d'espèces d'entérobactérie, ladite sonde comprenant un oligonucléotide tel que défini précédemment. Cette sonde de thérapie, capable de s'hybrider sur l'ARN messager et/ou sur l'ADN génomique desdites bactéries peut bloquer les phénomènes de traduction et/ou de transcription et/ou de réplication. Le principe des méthodes de thérapie génique est connu et repose notamment sur l'utilisation d'une sonde correspondant à un brin anti-sens : la formation d'un hybride entre la sonde et le brin sens est capable de perturber au moins l'une des étapes du décryptage de l'information génétique. Les sondes de thérapie sont donc utilisables comme médicaments antibactériens, permettant de lutter contre les infections causées par des d'entérobactéries.

Dans des conditions qui sont précisées dans l'exemple 1 ci-après, on a déterminé la séquence nucléotidique de l'ADN correspondant au gène rpoB de deux espèces d'entérobactérie, ainsi que d'une espèce appartenant à une famille de bactéries également Gram négatives. Cette étude a porté sur les espèces *Escherichia coli, Shigella dysenteriae* et *Pseudomonas putida*.

On a recherché des régions fortement conservées pour les seules espèces entérobactériennes, permettant de les distinguer des autres genres bactériens. On a ainsi sélectionné, en faisant référence à la numérotation de la séquence nucléotidique du gène rpoB de *Escherichia coli* ATCC 25290, une région intéressante de 512 bases, à savoir des positions 1498 à 2009 du gène rpoB. On a déterminé les séquences de ces mêmes régions pour d'autres espèces d'entérobactéries, ainsi que pour plusieurs espèces n'appartenant pas à la famille des entérobactéries.

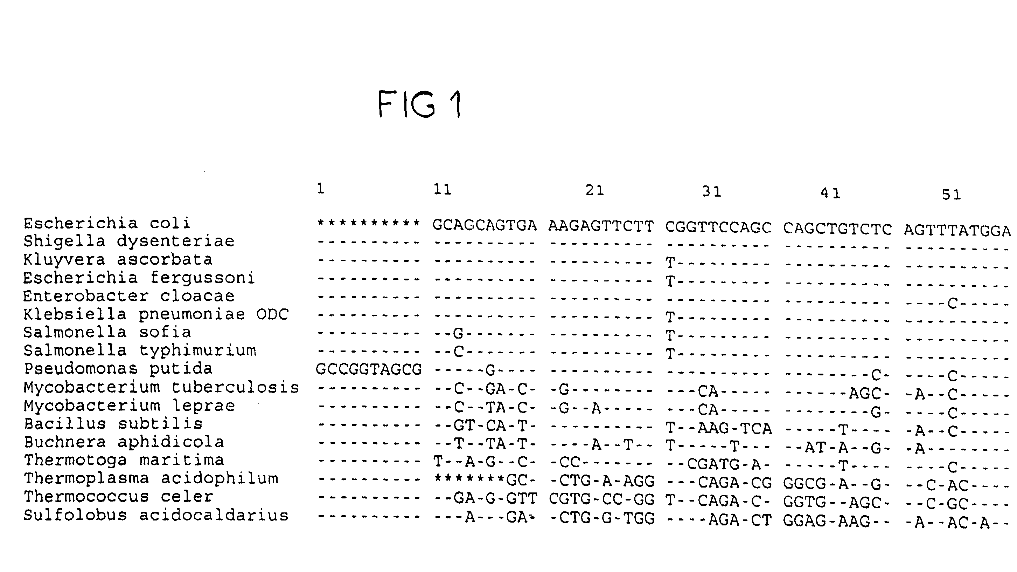

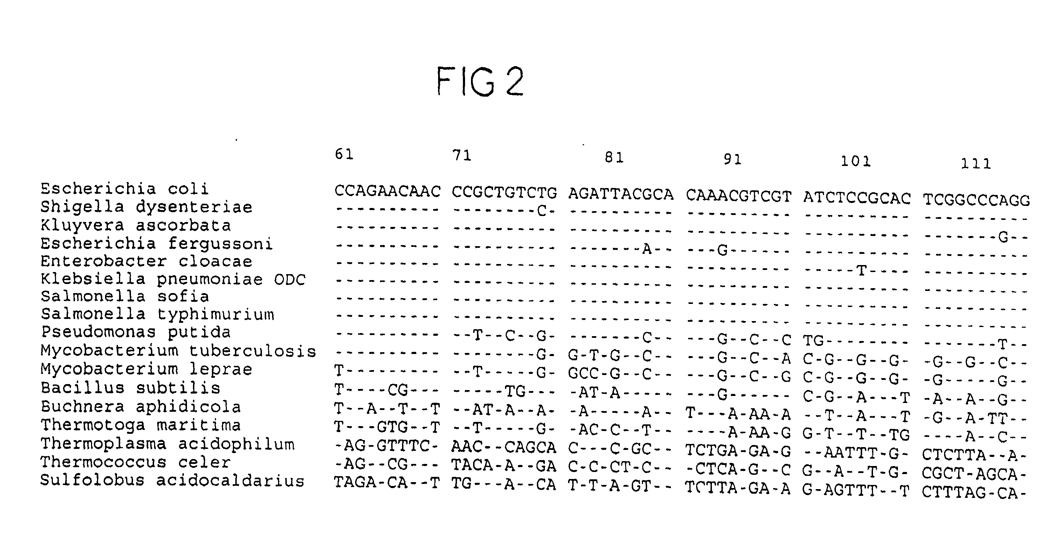

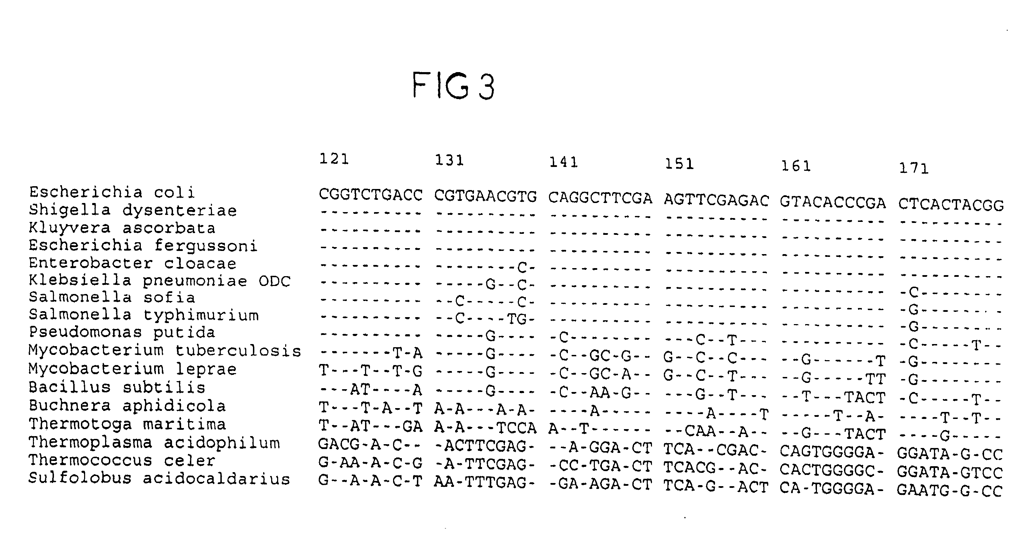

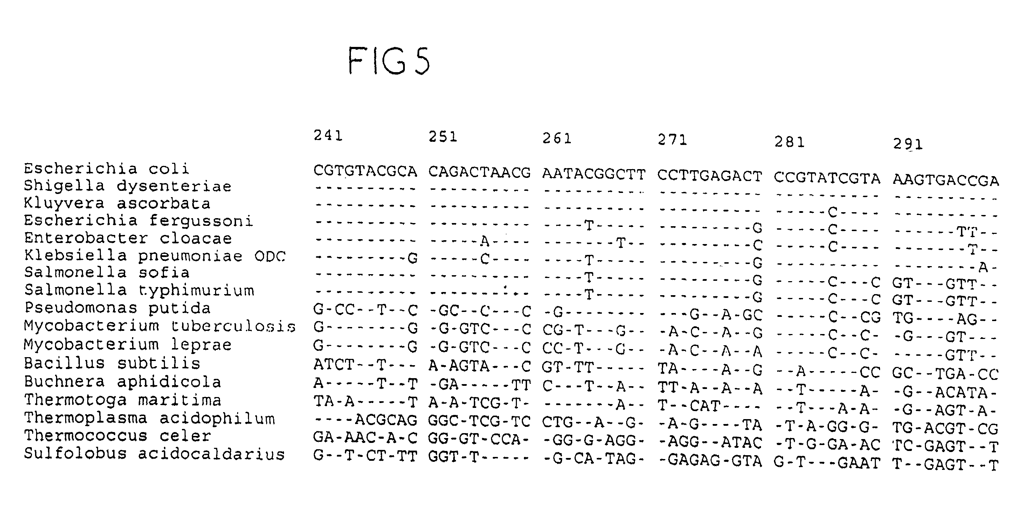

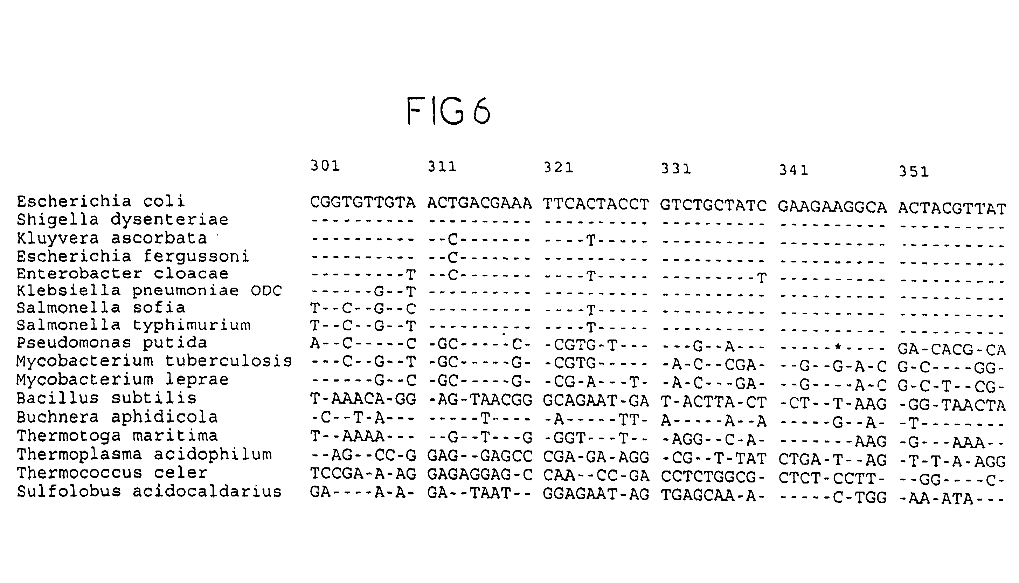

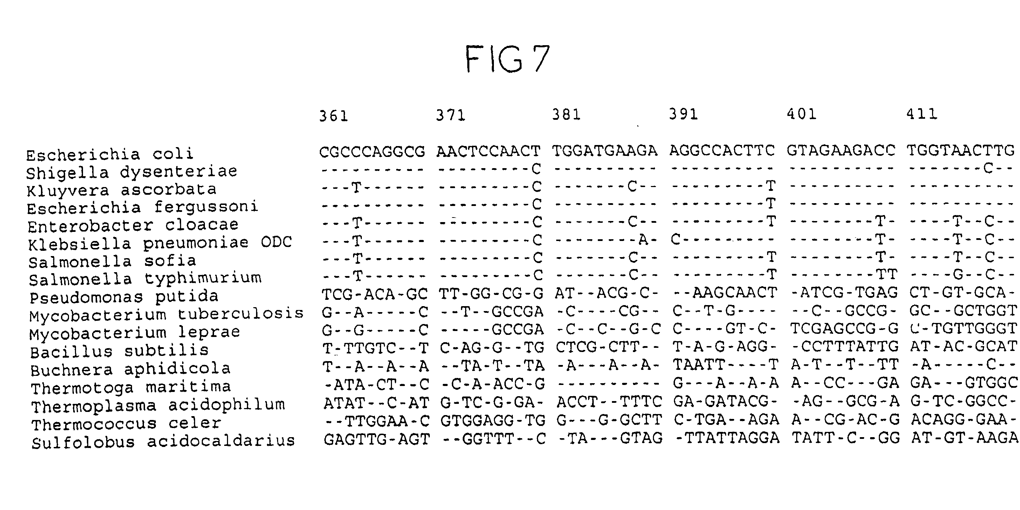

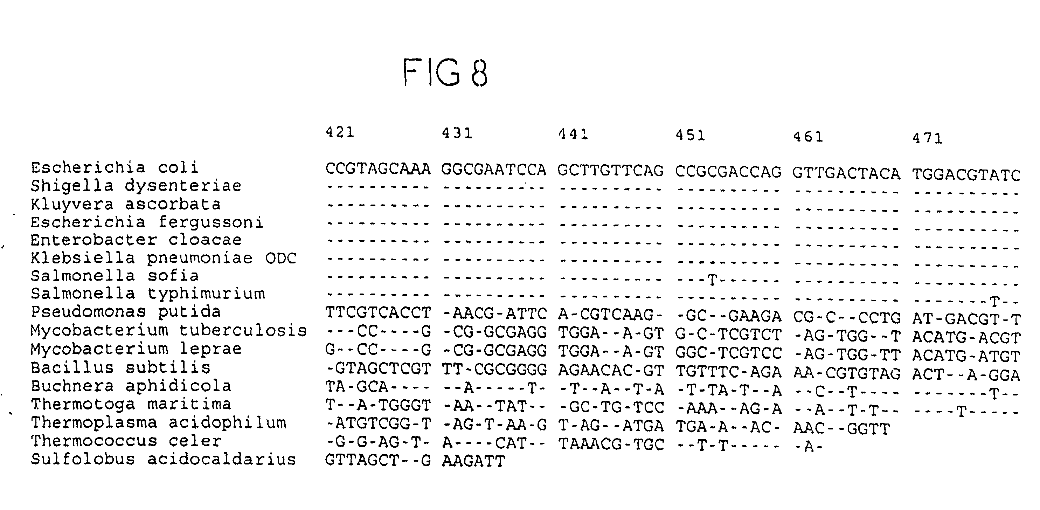

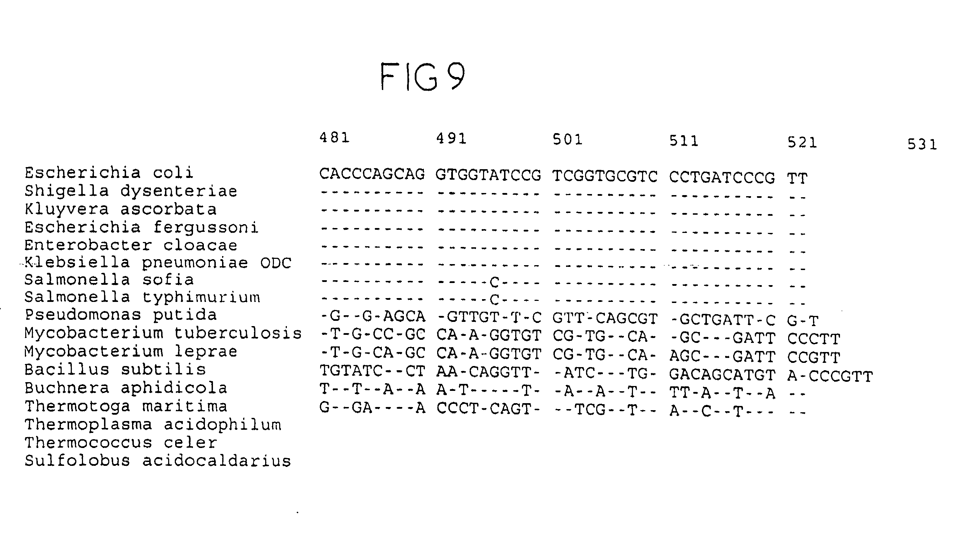

Les résultats de ces différents séquençages sont résumés dans les figures 1 à 9 annexées dans lesquelles le signe * représente un site vacant dont la représentation est nécessaire pour l'alignement des séquences en tenant compte de certaines espèces possédant à ce site un motif nucléotidique supplémentaire.

Les résultats de ces recherches, c'est-à-dire les alignements de séquences figurant dans les figures 1 à 9, ont permis de concevoir des sondes dans les régions conservées chez toutes les espèces d'entérobactéries, qui permettent de détecter la présence ou l'absence d'au moins une bactérie quelconque de la famille des entérobactéries. En outre, l'utilisation de sondes contenant des zones mutées pour une espèce particulière (par rapport à l'espèce de référence, ici *E. coli*), rend possible la détection directe d'une telle espèce. Dans les techniques d'hybridation sandwich, utilisant deux sondes en combinaison (sonde de capture et sonde de détection), on utilisera par exemple en combinaison une sonde spécifique de la famille des entérobactéries et une sonde spécifique de l'espèce considérée. On peut aussi utiliser en combinaison deux sondes spécifiques de ladite espèce, lorsqu'elles existent, ces deux sondes étant complémentaires de régions non chevauchantes du gène rpoB.

Comme on l'a indiqué précédemment, les sondes nucléotidiques de l'invention peuvent être utilisées dans les méthodes classiques d'hybridation. L'acide nucléique à détecter (cible) peut être de l'ADN ou de l'ARN (l'un ou l'autre éventuellement obtenu après amplification). Dans le cas d'une cible nucléique double brin, il convient de procéder à sa dénaturation avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80°C.

Dans certains cas, la mise en évidence de la présence d'une espèce d'Entérobacterie donnée nécessite l'utilisation de sondes permettant la détection d'une mutation ponctuelle. Pour cela, il convient d'opérer avec des sondes d'une longueur (nombre de motifs nucléotidiques) prédéterminée, dans des conditions elles-mêmes prédéterminées.

D'autres précisions sont données ci-après, en faisant référence plus particulièrement à la méthode d'hybridation sandwich qui constitue l'une des méthodes d'hybridation les plus pratiques actuellement. Cette méthode comprend la mise en contact d'une première sonde fixée sur un support solide avec une solution contenant l'acide nucléique à analyser, et la mise en contact dudit support avec la sonde de détection, l'incubation du mélange obtenu, le rinçage du support, pour éliminer les constituants non fixés sur le support par hybridation spécifique, et la détection qualitative ou quantitative, à l'aide d'une réaction de révélation du marqueur fixé sur le support, de la sonde de détection fixée. La révélation de la présence du marqueur peut être effectuée par exemple par colorimétrie, fluorescence ou luminescence.

La mise en contact de la sonde de capture avec l'échantillon et avec la sonde de détection peut être effectuée de façon séquentielle, éventuellement avec rinçage intermédiaire du support. On peut également opérer par mise en contact simultanée ou quasi-simultanée de la sonde de capture fixée sur le support avec une solution contenant l'échantillon et la sonde de détection qui peuvent être ajoutées sous forme de mélange ou séparément.

Les stades d'incubation et de lavage consécutif, qui constituent les étapes-clés du procédé d'hybridation sandwich, sont chacune effectuée à une température constante qui peut être par exemple comprise dans la gamme mentionnée plus haut (voir les "Définitions"). On sait que les hybrides d'acides nucléiques ont une température de dissociation qui dépend du nombre de bases hybridées (la température augmentant avec la taille de l'hybride) et qui dépend également de la nature des bases hybridées et, pour chaque base hybridée, de la nature des bases adjacentes. La dissociation des hybrides se produit sur un intervalle de température de quelques degrés et peut être facilement déterminée, par exemple en spectroscopie U.V.. Il est possible de déterminer expérimentalement la température de demi-dissociation de l'hybride formé par une sonde donnée avec la cible de séquence complémentaire, par de simples expériences de routine. La température d'hybridation utilisée dans la technique d'hybridation sandwich doit évidemment être choisie en-dessous de la température de demi-dissociation. Plus exactement, on opère à une température inférieure à la température de demi-dissociation de l'hybride le moins stable parmi les deux hybrides que forme la cible avec d'une part la sonde de capture et d'autre part la sonde de détection, afin que les deux hybrides soient stables à la température à laquelle on opère. Une mutation ponctuelle, c'est-à-dire une mutation entraînant un mésappariement portant sur une seule paire de bases dans l'hybride, entraîne une modification, généralement un abaissement, de la température de demi-dissociation. En utilisant des sondes suffisamment courtes, un tel mésappariement unique peut entraîner un abaissement relativement important de la température de demi-dissociation, de l'ordre de quelques degrés. Ainsi, en choisissant, à l'aide d'expériences de routine préalables, une sonde de longueur appropriée, en opérant dans une solution tampon donnée, il est possible de déterminer une température à laquelle il sera possible de n'observer l'hybridation que dans le cas où la sonde est strictement complémentaire de la cible. En outre, grâce au choix de sondes courtes de longueur prédéterminée, il est possible de mettre en oeuvre la technique d'hybridation sandwich à une température unique prédéterminée, par exemple 37°C. Pour une discussion plus détaillée de la technique d'hybridation sandwich avec utilisation de sondes courtes, on peut se reporter notamment à la demande de brevet FR-2 663 040.

Les exemples suivants illustrent l'invention.

### EXEMPLE 1

Des amorces conservées ont été sélectionnées pour amplifier la partie séquencée : CM7: 5' AACCAGTTCCGCGTTGGCCTGG 1348 -1405 CM31 5' CCTGAACAACACGCTCGGA 2473-2455 les coordonnées étant exprimées selon la séquence codante du gène rpoB d'E. coli V00340 de Genbank.

L'ADN cible est extrait de chaque bactérie selon la technique suivante. 1,5 ml de culture bactérienne est centrifugé et le culot repris dans 75 µl de tampon Tris (Tris 25 mM, EDTA 10 mM, saccharose 50 mM pH 8) et 25 µl de solution de lysozyme (1 mg/ml en tampon Tris). Aprés incubation 30 min à 37°C, 50 µl de phénol sont ajoutés et le tube est agité fortement pendant 30 secondes. On rajoute 50 µl de chloroforme et on agite à nouveau 2 secondes. Après centrifugation pendant 5 min, on récupère la phase aqueuse.

L'ADN est amplifié selon la technique PCR de Saiki et al. (Saiki et al. 1988. Science 239, 487-494) à l'aide d'un appareil PCR 480 Perkin-Elmer. Le milieu de réaction (100 µl) est constitué de Tris.HCl, 10mmol/l; MgCl₂ 1,5 mmol/l; KCl 50 mmol/l; gélatine 1 mg/ml; dATP, dCTP, dGTP, dTTP 0,5 mmol/l chacun; pH 8,3; oligonucléotide CM7, 25 pmol; oligonucléotide CM31, 25 pmol et 10 µl de la préparation d'ADN bactérien. Après dénaturation 5 mn suivie d'une centrifugation, on ajoute l'enzyme à 1,5 U/réaction. La PCR est effectuée sur 30 cycles avec les paramêtres 96°C/60°C/74°C, pendant 1 mn, 1 mn, et 0,7 mn respectivement.

L'ADN amplifié est analysé par electrophorèse en gel d'agarose 0,8% en tampon TBE (89 mM Tris base, 89 mM acide borique, 2 mM EDTA). Les bandes sont visualisées par le bromure d'éthidium.

La séquence des fragments ainsi amplifiés a été établie selon les techniques classiques.

### EXEMPLE 2 : Utilisation d'une sonde spécifique dirigée contre le gène rpoB pour l'identification d'Escherichia coli

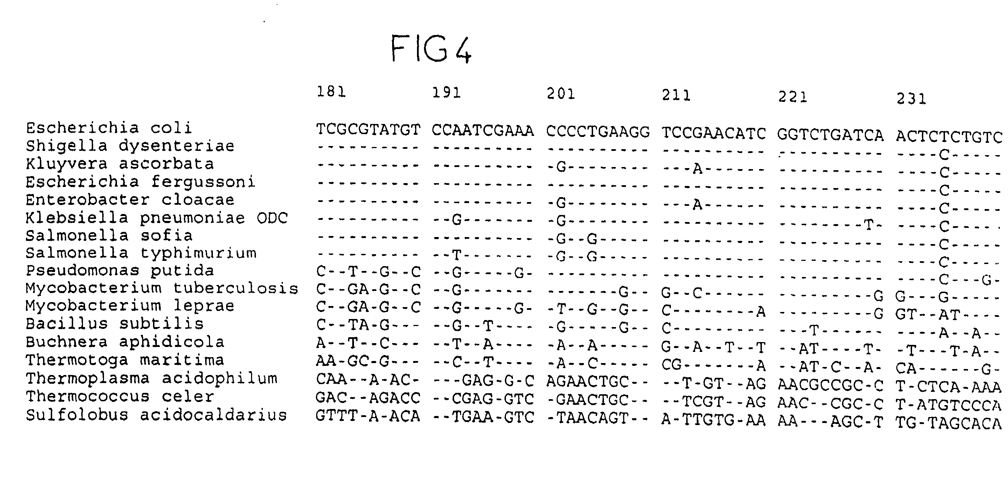

La sonde commençant au nucléotide N°4 et finissant au nucléotide N°27 de la séquence SEQ ID NO12, a priori spécifique pour les souches d'*E. coli* a été déduite d'après les alignements des séquences nucléotidiques des gènes rpoB de la figure 4. Une collection de souches bactériennes a été testée par hybridation sur le fragment amplifié à partir de rpoB et a permis de constater a posteriori la spécificité de cette sonde.

L'hybridation du produit PCR d'une bactérie-cible a été conduite selon le procédé de détection non-radioactif et semi-automatisé décrit dans le brevet français n°90 07249. Une sonde de capture S8L correspondant à une sonde de spécificité eubactérienne (décrite dans la demande de brevet FR n°93 02127) et un conjugué spécifique de détection oligonucléotide (correspondant à la sonde spécifique définie plus haut ) - enzyme sont utilisés. La manipulation a été conduite selon le protocole suivant.

Dans une plaque de microtitration (Nunc 439454) est déposée une solution de l'oligonucléotide de capture à 1 ng/ml dans du PBS 3X (0,45 M NaCl 0,15 M phosphate de sodium pH 7.0) dont l'extrémité 5' a réagi avec le réactif Aminolink 2 (Applied Biosystems, Foster city, Californie). La plaque est incubée 2 h à 37° C puis lavée 3 fois avec 300 µl de PBST (PBS 1x, Tween 20: 0,5 °/00 (Merck 822184)). Le réactif Aminolink 2 permet d'ajouter à l'extrémité 5' de la sonde un bras comportant un groupement 6-aminohexyle. La sonde ainsi couplée à un ligand possédant un groupement polaire (amine primaire), et fixée de façon passive sur le support, procure un signal amélioré; voir la demande de brevet FR 91 09057.

La cible constituée par 4 µl du produit amplifié est mélangée avec 76 µl de tampon PBS saumon (PBS3X + ADN de sperme de saumon 10 µg/ml (Sigma D9156)) et 10 µl de soude 2N. L'ensemble est neutralisé 5 min aprés par l'addition de 10 µl d'acide acétique 2N. L'ensemble est ajouté dans le puits en plus de 50 µl d'une solution du conjugué oligonucléotide-péroxydase à la concentration de 0,1 ng/ml en oligonucléotide dans un tampon PBS cheval (PBS3X + 10% sérum de cheval (BioMérieux 55842)). Le conjugué oligonucléotide-péroxydase constitue la sonde de détection. La plaque est incubée 1 h à 37° C et lavée par 3 X 300 µl de PBST. Dans chaque puits, on rajoute 100 µl de substrat OPD (ortho-phenylenediamine Cambridge Medical Biotechnology ref/456) dans un tampon adapté (0,055 M acide citrique, 0,1 M Na₂HPO₄, pH 4,93) à la concentration de 4 mg/ml auquel on ajoute extemporanément H₂O₂ à 30 volumes au 1/1000. Après 20 mn de réaction l'activité enzymatique est bloquée par 100 µl d'H₂SO₄ 1N et la lecture est effectuée sur Axia Microreader (BioMérieux) à 492 nm.

Les bactéries-cibles étaient notamment les suivantes :
- 22 isolats ou souches *E. coli* (dont ATCC 25290, 25922, 27165, 10536);
- Divers autres Escherichia (18 isolats) : : *E. fergusonii, E. hermanii, E. vulneris* ;
- 8 Shigella : *S. boydii, S. dysenteriae* (dont ATCC 29027), *S. flexneri, S. sonnei* ;
- 5 Salmonella : S. *arizonae* (ATCC 13314), S. *choleraesuis* (ATCC 13312), *S. gallinarum, S. typhimurium (ATCC 14028), S. spp* (ATCC 9712) ;
- 7 entérobactéries diverses: *Citrobacter amalonaticus* (ATCC 25405), *Enterobacter cloacae* (ATCC 13047), *Klebsiella oxytoca, K. pneumoniae, Kluivera ascorbata, Proteus mirabilis, Serratia marcescens* (ATCC 8195) ;
- *Pseudomonas aeruginosa* ATCC 35422, *Pseudomonas putida ;*
- *Enterococcus faecalis.*

Les résultats obtenus indiquent que la combinaison de sondes utilisée est, a posteriori, spécifique d'*E*. *coli*. Elle ne présente pas de réactions croisées avec le produit PCR des autres espèces bactériennes.

L'adaptation de la combinaison spécifique sur l'automate VIDAS (marque déposée - commercialisé par la société bioMérieux-VITEK) a été effectuée. La paroi du puits de microplaque est ici remplacée par le SPR (marque de commerce) ("Solid Phase Réceptacle") qui est un support conique réalisé à partir d'un matériau vendu sous la dénomination K résine (copolymère butadiène-styrène) et commercialisé par la société bioMérieux-VITEK (USA). Les divers réactifs sont disposés dans une barrette à plusieurs cuvettes et les différentes étapes se déroulent dans le SPR qui est capable d'aspirer et de refouler les réactifs et qui fait donc office de pipette. La réaction d'hybridation sandwich décrite dans le protocole ci-dessous se produit sur la paroi interne du cône.

Sur la surface interne du SPR, est fixé passivement l'oligonucléotide de capture comportant à son extrémité 5' le ligand Aminolink 2 (Applied Biosystems-réf.400808) à une concentration de 1 ng/µl dans un volume de 315 µl d'une solution de PBS 4x (phosphate de sodium 200 mM pH 7,0, NaCl 600 mM). Après une nuit à température ambiante ou deux heures à 37°C, les cônes sont lavés 2 fois par une solution PBS Tween, puis séchés sous vide. La barrette contient dans des cuvettes les réactifs nécessaires à la détection, c'est à dire: 200 µl d'une solution à 0,1 ng/µl du conjugué de détection oligonucléotide-phosphatase alcaline, 2 fois 600 µl de solution de lavage PBS Tween et une cuvette de substrat.

Dans le premier puits de la barrette, sont déposés 10 µl de produit PCR dans le même tampon que pour le protocole microplaque ci-dessus.

Après incubation 30 minutes du cône par le mélange cible PCR plus sonde de détection, le cône est lavé 2 fois par une solution de PBS Tween. 250 µl de substrat MUP (méthyl-4-umbelliféryl phosphate) en solution dans un tampon diéthanolamine sont aspirés dans le cône, puis relachés dans une cuvette de lecture. L'appareil mesure le signal fluorescent exprimé en URF (unités relatives de fluorescence) de la cuvette.

Les résultats obtenus avec ce système sont les mêmes que ceux obtenus en microplaque.
1 INFORMATIONS GENERALES:
   (i) DEPOSANT:
      (A) NOM: BIO MERIEUX
      (B) RUE: Chemin de l'Orme
      (C) VILLE: MARCY L'ETOILE
      (D) ETAT OU PROVINCE:
      (E) PAYS: FRANCE
      (F) CODE POSTAL: 69280
   (ii) TITRE DE L'INVENTION: Détection des Entérobactéries
   (iii) NOMBRE DE SEQUENCES: 79
   (iv) ADRESSE DE CORRESPONDANCE:
      (A) NOM: Cabinet GERMAIN & MAUREAU
      (B) RUE: 20 Bd Eugène Deruelle
      (C) VILLE: LYON
      (D) PAYS: FRANCE
      (E) CODE POSTAL: 69003
      (F) TELEPHONE: 72 60 28 90
      (G) TELEFAX: 78 60 92 85
   (v) FORME DECHIFFRABLE PAR ORDINATEUR:
      (A) TYPE DE SUPPORT: disquette 3,5 pouces DS, HD
      (B) ORDINATEUR: EPSON (compatible IBM)
      (C) SYSTEME D'EXPLOITATION: DOS
      (D) LOGICIEL: MICROSOFT WORD POUR WINDOWS
   (vi) DEMANDE ACTUELLE:
      (A) DATE DE LA DEMANDE:
      (B) NUMERO DE LA DEMANDE:
   (vii) DATE DE LA DEMANDE ANTERIEURE: 18.09.1995
      (A) NUMERO DE LA DEMANDE: 95 11125
      (B) CLASSIFICATION:
   (viii) MANDATAIRE:
      (A) NOM: CABINET GERMAIN & MAUREAU, Dominique GUERRE
      (B) REFERENCE DOSSIER: MD/B 05 B 2333
         NOMBRE DE SEQUENCES : 79
2 INFORMATIONS POUR LA SEQ ID NO : 1
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION : linéaire
   2ii TYPE DE MOLECULE :
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO : 1 GCAGTGAAAG AGTTCTT 17
2 INFORMATIONS POUR LA SEQ ID NO : 2
   2i CARACTERISTIQUES DE LA SEQUENCE :
      2iA LONGUEUR : 31 paires de bases
      2iB TYPE : acide nucléique
      2iC NOMBRE DE BRINS : simple
      2iD CONFIGURATION : linéaire
   2ii TYPE DE MOLECULE : ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 2 
SEQ ID NO2 :
CAGGCTTCGA AGTTCGAGAA CGTACACCCG A 31
2 INFORMATIONS POUR LA SEQ ID NO : 3
   2i CARACTERISTIQUES DE LA SEQUENCE :
      2iA LONGUEUR : 38 paires de bases
      2iB TYPE : acide nucléique
      2iC NOMBRE DE BRINS : simple
      2iD CONFIGURATION : linéaire
   2ii TYPE DE MOLECULE : ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 3 
SEQ ID NO3 :
TACCTGTCTG CTATYGAAGA AGGCAACTAC GTTATCGC 38
2 INFORMATIONS POUR LA SEQ ID NO : 4
   2i CARACTERISTIQUES DE LA SEQUENCE :
      2iA LONGUEUR : 102 paires de bases
      2iB TYPE : acide nucléique
      2iC NOMBRE DE BRINS : simple
      2iD CONFIGURATION : linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 4 
SEQ ID NO4 :

TGCCGTAGCA AAGGCGAATC CAGCTTGTTC AGCCGYGACC AGGTTGACTA CATGGACGTW

TCCACCCAGC AGGTGGTMTC CGTCGGTGCG TCCCTGATCC CG 102
2 INFORMATIONS POUR LA SEQ ID NO : 5
   2i CARACTERISTIQUES DE LA SEQUENCE :
      2iA LONGUEUR : 20 paires de bases
      2iB TYPE : acide nucléique
      2iC NOMBRE DE BRINS : simple
      2iD CONFIGURATION : linéaire
   2ii TYPE DE MOLECULE : ADNc
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 5 
SEQ ID NO5 :
GCGGCAGTGA AAGAGTTCTT 20
2 INFORMATIONS POUR LA SEQ ID NO : 6
   2i CARACTERISTIQUES DE LA SEQUENCE :
      2iA LONGUEUR : 20 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 6 GCCGCAGTGA AAGAGTTCTT 20
2 INFORMATIONS POUR LA SEQ ID NO : 7
   2i CARACTERISTIQUES DE LA SEQUENCE :
      2iA LONGUEUR : 34 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 7 GAACAACCCG CTGTCCGAGA TTACGCACAA ACGT 34
2 INFORMATIONS POUR LA SEQ ID NO : 8
   2i CARACTERISTIQUES DE LA SEQUENCE :
      2iA LONGUEUR : 33 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 8 TCTGAGATTA CACACAAGCG TCGTATCTCC GCA 33
2 INFORMATIONS POUR LA SEQ ID NO : 9
   2i CARACTERISTIQUES DE LA SEQUENCE :
      2iA LONGUEUR : 30 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 9 CAAACGTCGT ATCTCTGCAC TCGGCCCAGG 30
2 INFORMATIONS POUR LA SEQ ID NO : 10
   2i CARACTERISTIQUES DE LA SEQUENCE :
      2iA LONGUEUR : 32 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 10 TCTGACCCGT GAGCGCGCAG GCTTCGAAGT TC 32
2 INFORMATIONS POUR LA SEQ ID NO : 11
   2i CARACTERISTIQUES DE LA SEQUENCE :
      2iA LONGUEUR : 27 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 11 TCTGACCCGC GAACTGGCAG GCTTCGA 27
2 INFORMATIONS POUR LA SEQ ID NO : 12
   2i CARACTERISTIQUES DE LA SEQUENCE :
      2iA LONGUEUR : 30 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 12 GGTCTGATCA ACTCTCTGTC CGTGTACGCA 30
2 INFORMATIONS POUR LA SEQ ID NO : 13
   2i CARACTERISTIQUES DE LA SEQUENCE :
      2iA LONGUEUR : 29 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 13 GTGTACGCAC AGACAAACGA ATACGGCTT 29
2 INFORMATIONS POUR LA SEQ ID NO : 14
   2i CARACTERISTIQUES DE LA SEQUENCE :
      2iA LONGUEUR : 27 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 14 GTCCGTGTAC GCGCAGACCA ACGAATA 27
2 INFORMATIONS POUR LA SEQ ID NO : 15
   2i CARACTERISTIQUES DE LA SEQUENCE :
      2iA LONGUEUR : 18 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 15 CGCGCAGACC AACGAATA 18
2 INFORMATIONS POUR LA SEQ ID NO : 16
   2i CARACTERISTIQUES DE LA SEQUENCE :
      2iA LONGUEUR : 25 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 16 CAAACGAATA CGGTTTCCTT GAGAC 25
2 INFORMATIONS POUR LA SEQ ID NO : 17
   2i CARACTERISTIQUES DE LA SEQUENCE :
      2iA LONGUEUR : 15 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 17 CCTTGAGACC CCGTA 15
2 INFORMATIONS POUR LA SEQ ID NO : 18
   2i CARACTERISTIQUES DE LA SEQUENCE :
      2iA LONGUEUR : 23 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 18 CGTAAAGTGA CTGACGGTGT TGT 23
2 INFORMATIONS POUR LA SEQ ID NO : 19
   2i CARACTERISTIQUES DE LA SEQUENCE :
      2iA LONGUEUR : 23 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 19 CGTAAAGTGA TTGACGGTGT TGT 23
2 INFORMATIONS POUR LA SEQ ID NO : 20
   2i CARACTERISTIQUES DE LA SEQUENCE :
      2iA LONGUEUR : 20 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 20 CGTAAAGTGA CCAACGGTGT 20
2 INFORMATIONS POUR LA SEQ ID NO : 21
   2i CARACTERISTIQUES DE LA SEQUENCE :
      2iA LONGUEUR : 22 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 21 CGGTGTTGTT ACCGACGAAA TT 22
2 INFORMATIONS POUR LA SEQ ID NO : 22
   2i CARACTERISTIQUES DE LA SEQUENCE :
      2iA LONGUEUR : 30 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 22 TACCTGTCTG CTATTGAAGA AGGCAACTAC 30
2 INFORMATIONS POUR LA SEQ ID NO : 23
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 23 TGGATGAAAA CGGCCACTT 19
2 INFORMATIONS POUR LA SEQ ID NO : 24
   2i CARACTERISTIQUES DE LA SEQUENCE :
      2iA LONGUEUR : 14 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 24 GTAGAAGATT TGGT 14
2 INFORMATIONS POUR LA SEQ ID NO : 25
   2i CARACTERISTIQUES DE LA SEQUENCE :
      2iA LONGUEUR : 22 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 25 TGGTGACCTG CCGTAGCAAA GG 22
2 INFORMATIONS POUR LA SEQ ID NO : 26
   2i CARACTERISTIQUES DE LA SEQUENCE :
      2iA LONGUEUR : 23 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 26 CAGGCGAACT CCAACTTGGA TGA 23
2 INFORMATIONS POUR LA SEQ ID NO : 27
   2i CARACTERISTIQUES DE LA SEQUENCE :
      2iA LONGUEUR : 30 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 27 CAGCTTGTTC   AGCCGTGACC   AGGTTGACTA   30
2 INFORMATIONS POUR LA SEQ ID NO : 28
   2i CARACTERISTIQUES DE LA SEQUENCE :
      2iA LONGUEUR : 27 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 28 ACATGGACGT TTCCACCCAG CAGGTGG 27
2 INFORMATIONS POUR LA SEQ ID NO : 29
   2i CARACTERISTIQUES DE LA SEQUENCE :
      2iA LONGUEUR : 15 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 29 GCGGCAGTGA AAGAG 15
2 INFORMATIONS POUR LA SEQ ID NO : 30
   2i CARACTERISTIQUES DE LA SEQUENCE :
      2iA LONGUEUR : 15 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 30 GCCGCAGTGA AAGAG 15
2 INFORMATIONS POUR LA SEQ ID NO : 31
   2i CARACTERISTIQUES DE LA SEQUENCE :
      2iA LONGUEUR : 23 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 31 AACCCGCTGT CCGAGATTAC GCA 23
2 INFORMATIONS POUR LA SEQ ID NO : 32
   2i CARACTERISTIQUES DE LA SEQUENCE :
      2iA LONGUEUR : 16 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 32 TACACACAAG CGTCGT 16
2 INFORMATIONS POUR LA SEQ ID NO : 33
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 33 CGTATCTCTG CACTCGG 17
2 INFORMATIONS POUR LA SEQ ID NO : 34
   2i CARACTERISTIQUES DE LA SEQUENCE :
      2iA LONGUEUR : 18 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 34 CGTGAGCGCG CAGGCTTC 18
2 INFORMATIONS POUR LA SEQ ID NO : 35
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 35 CCGCGAACTG GCAGGCTTC 19
2 INFORMATIONS POUR LA SEQ ID NO : 36
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 36 GATCAACTCT CTGTCCGTG 19
2 INFORMATIONS POUR LA SEQ ID NO : 37
   2i CARACTERISTIQUES DE LA SEQUENCE :
      2iA LONGUEUR : 18 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 37 GCACAGACAA ACGAATAC 18
2 INFORMATIONS POUR LA SEQ ID NO : 38
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 38 GTGTACGCGC AGACTCAAC 19
2 INFORMATIONS POUR LA SEQ ID NO : 39
   2i CARACTERISTIQUES DE LA SEQUENCE :
      2iA LONGUEUR : 14 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 39 CGCGCAGACC AACG 14
2 INFORMATIONS POUR LA SEQ ID NO : 40
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 40 GGAATACGGT TTCCTTG 17
2 INFORMATIONS POUR LA SEQ ID NO : 41
   2i CARACTERISTIQUES DE LA SEQUENCE :
      2iA LONGUEUR : 15 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 41 CCTTGAGACC CCGTA 15
2 INFORMATIONS POUR LA SEQ ID NO : 42
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 42 CGTAAAGTGA CTGACGG 17
2 INFORMATIONS POUR LA SEQ ID NO : 43
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 43 CGTAAAGTGA TTGACGG 17
2 INFORMATIONS POUR LA SEQ ID NO : 44
   2i CARACTERISTIQUES DE LA SEQUENCE :
      2iA LONGUEUR : 18 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 44 GTAAAGTGAC CAACGGTG 18
2 INFORMATIONS POUR LA SEQ ID NO : 45
   2i CARACTERISTIQUES DE LA SEQUENCE :
      2iA LONGUEUR : 18 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 45 GTGTTGTTAC CGACGAAA 18
2 INFORMATIONS POUR LA SEQ ID NO : 46
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 46 GTCTGCTATT GAAGAAGGC 19
2 INFORMATIONS POUR LA SEQ ID NO : 47
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 47 TGGATGAAAA CGGCCACTT 19
2 INFORMATIONS POUR LA SEQ ID NO : 48
   2i CARACTERISTIQUES DE LA SEQUENCE :
      2iA LONGUEUR : 14 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 48 GTAGAAGATT TGGT 14
2 INFORMATIONS POUR LA SEQ ID NO : 49
   2i CARACTERISTIQUES DE LA SEQUENCE :
      2iA LONGUEUR : 15 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 49 TGGTGACCTG CCGTA 15
2 INFORMATIONS POUR LA SEQ ID NO : 50
   2i CARACTERISTIQUES DE LA SEQUENCE :
      2iA LONGUEUR : 19 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 50 CGAACTCCAA CTTGGATGA 19
2 INFORMATIONS POUR LA SEQ ID NO : 51
   2i CARACTERISTIQUES DE LA SEQUENCE :
      2iA LONGUEUR : 20 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 51 GTTCAGCCGT GACCAGGTTG 20
2 INFORMATIONS POUR LA SEQ ID NO : 52
   2i CARACTERISTIQUES DE LA SEQUENCE :
      2iA LONGUEUR : 17 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 52 GGACGTTTCC ACCCAGC 17
2 INFORMATIONS POUR LA SEQ ID NO : 53
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 53 GGCAG 5
2 INFORMATIONS POUR LA SEQ ID NO : 54
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 54 CGCAG 5
2 INFORMATIONS POUR LA SEQ ID NO : 55
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 55 TGTCCGAGA 9
2 INFORMATIONS POUR LA SEQ ID NO : 56
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 56 ACACA 5
2 INFORMATIONS POUR LA SEQ ID NO : 57
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 57 GCGTC 5
2 INFORMATIONS POUR LA SEQ ID NO : 58
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 58 TCTCTGCAC 9
2 INFORMATIONS POUR LA SEQ ID NO : 59
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 59 AGCGCGCAG 9
2 INFORMATIONS POUR LA SEQ ID NO : 60
   2i CARACTERISTIQUES DE LA SEQUENCE :
      2iA LONGUEUR : 10 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 60 GAACTGGCAG 10
2 INFORMATIONS POUR LA SEQ ID NO : 61
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 61 ACTCTCTGT 9
2 INFORMATIONS POUR LA SEQ ID NO : 62
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 62 AGACAAACG 9
2 INFORMATIONS POUR LA SEQ ID NO : 63
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 63 GCAGA 5
2 INFORMATIONS POUR LA SEQ ID NO : 64
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 64 AGACC 5
2 INFORMATIONS POUR LA SEQ ID NO : 65
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 65 ACGGTTTCC 9
2 INFORMATIONS POUR LA SEQ ID NO : 66
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 66 TGACT 5
2 INFORMATIONS POUR LA SEQ ID NO : 67
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 67 TGATT 5
2 INFORMATIONS POUR LA SEQ ID NO : 68
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 68 GACCA 5
2 INFORMATIONS POUR LA SEQ ID NO : 69
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 69 TTACC 5
2 INFORMATIONS POUR LA SEQ ID NO : 70
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 70 CTATTGAAG 9
2 INFORMATIONS POUR LA SEQ ID NO : 71
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 71 AAACG 5
2 INFORMATIONS POUR LA SEQ ID NO : 72
   2i CARACTERISTIQUES DE LA SEQUENCE :
      2iA LONGUEUR : 8 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 72 AGATTTGG 8
2 INFORMATIONS POUR LA SEQ ID NO : 73
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 73 TGACC 5
2 INFORMATIONS POUR LA SEQ ID NO : 74
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 74 CAACTTGGA 9
2 INFORMATIONS POUR LA SEQ ID NO : 75
   2i CARACTERISTIQUES DE LA SEQUENCE :
      2iA LONGUEUR : 9 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 75 GCCGTGACC 9
2 INFORMATIONS POUR LA SEQ ID NO : 76
   2i CARACTERISTIQUES DE LA SEQUENCE :
      2iA LONGUEUR : 5 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 76 TTCCA 5
2 INFORMATIONS POUR LA SEQ ID NO : 77
   2i CARACTERISTIQUES DE LA SEQUENCE :
      2iA LONGUEUR : 30 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 77 CTAGGATCCT TAGAATTCAA CCCGCTGTCC 30
2 INFORMATIONS POUR LA SEQ ID NO : 78
   2i CARACTERISTIQUES DE LA SEQUENCE :
      2iA LONGUEUR : 21 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 78 TACAACTGGC AGGCTGCTTG A 21
2 INFORMATIONS POUR LA SEQ ID NO : 79
   2i CARACTERISTIQUES DE LA SEQUENCE :
      2iA LONGUEUR : 36 paires de bases
      2iB TYPE : acide nucléique
      2iC NOMBRE DE BRINS : simple
      2iD CONFIGURATION: linéaire
   2ii TYPE DE MOLECULE: ADN
   2iii HYPOTHETIQUE : non
   2iv ANTI-SENS :
   2v TYPE DE FRAGMENT :
   2vi ORIGINE : oligonucléotide de synthèse
   2xi DESCRIPTION DE LA SEQUENCE : SEQ ID NO: 79

TGTGCCAGCG GTACTGGACA GACCAACGAA AGATTA 36

## Revendications

1. Oligonucléotide choisi parmi les oligonucléotides comprenant une séquence d'au moins 12 nucléotides naturels ou modifiés consécutifs incluse dans l'une des séquences SEQ ID NO 1 à SEQ ID NO 52, et parmi les oligonucléotides complémentaires de ces oligonucléotides, à l'exclusion des oligonucléotides ayant une séquence choisie parmi les séquences suivantes : SEQ ID NO 28, SEQ ID NO 77, SEQ ID NO 78 et SEQ ID NO 79.

2. Oligonucléotide selon la revendication 1, **caractérisé en ce qu'**il comprend une séquence nucléotidique d'au moins 12 nucléotides naturels ou modifiés consécutifs incluse dans l'une des séquences SEQ ID NO1 à SEQ ID N04.

3. Oligonucléotide selon la revendication 1, **caractérisé en ce qu'**il comprend une séquence nucléotidique d'au moins 12 nucléotides naturels ou modifiés consécutifs incluse dans :
SEQ ID NO5 et contenant au moins 5 nucléotides naturels ou modifiés consécutifs inclus dans SEQ ID NO53,
SEQ ID NO6 et contenant au moins 5 nucléotides naturels ou modifiés consécutifs inclus dans SEQ ID NO54,
SEQ ID NO8 et contenant au moins 5 nucléotides naturels ou modifiés consécutifs inclus dans SEQ ID NO56,
SEQ ID NO8 et contenant au moins 5 nucléotides naturels ou modifiés consécutifs inclus dans SEQ ID NO57,
SEQ ID NO9 et contenant au moins 5 nucléotides naturels ou modifiés consécutifs inclus dans SEQ ID NO58,
SEQ ID NO10 et contenant au moins 5 nucléotides naturels ou modifiés consécutifs inclus dans SEQ ID NO59,
SEQ ID NO12 et contenant au moins 5 nucléotides naturels ou modifiés consécutifs inclus dans SEQ ID NO61,
SEQ ID NO13 et contenant au moins 5 nucléotides naturels ou modifiés consécutifs inclus dans SEQ ID NO62,
SEQ ID NO14 et contenant au moins 5 nucléotides naturels ou modifiés consécutifs inclus dans SEQ ID NO63,
SEQ ID NO16 et contenant au moins 5 nucléotides naturels ou modifiés consécutifs inclus dans SEQ ID NO65,
SEQ ID NO17 et contenant au moins 5 nucléotides naturels ou modifiés consécutifs inclus dans SEQ ID NO64,
SEQ ID NO18 et contenant au moins 5 nucléotides naturels ou modifiés consécutifs inclus dans SEQ ID NO66,
SEQ ID NO19 et contenant au moins 5 nucléotides naturels ou modifiés consécutifs inclus dans SEQ ID NO67,
SEQ ID NO20 et contenant au moins 5 nucléotides naturels ou modifiés consécutifs inclus dans SEQ ID NO68,
SEQ ID NO21 et contenant au moins 5 nucléotides naturels ou modifiés consécutifs inclus dans SEQ ID NO69,
SEQ ID NO22 et contenant au moins 5 nucléotides naturels ou modifiés consécutifs inclus dans SEQ ID NO70,
SEQ ID NO23 et contenant au moins 5 nucléotides naturels ou modifiés consécutifs inclus dans SEQ ID NO71,
SEQ ID NO24 et contenant au moins 5 nucléotides naturels ou modifiés consécutifs inclus dans SEQ ID NO72,
SEQ ID NO25 et contenant au moins 5 nucléotides naturels ou modifiés consécutifs inclus dans SEQ ID NO73,
SEQ ID NO26 et contenant au moins 5 nucléotides naturels ou modifiés consécutifs inclus dans SEQ ID NO74,
SEQ ID NO27 et contenant au moins 5 nucléotides naturels ou modifiés consécutifs inclus dans SEQ ID NO75.

4. Oligonucléotide selon la revendication 3, **caractérisé en ce qu'**il comprend ou consiste en une séquence nucléotidique choisie parmi les séquences SEQ ID NO29 à SEQ ID NO52.

5. Sonde pour la détection, dans un échantillon biologique, de bactéries appartenant à la famille des entérobactéries, consistant en un oligonucléotide selon la revendication 2.

6. Sonde pour la détection, dans un échantillon biologique, d'au moins une espèce d'entérobactéries, consistant en un oligonucléotide selon la revendication 3 ou 4.

7. Sonde selon la revendication 5 ou 6, **caractérisée en ce qu'**elle est immobilisée sur un support solide.

8. Sonde selon l'une quelconque des revendications 5 à 7, **caractérisée par le fait qu'**elle est marquée avec un agent traceur.

9. Utilisation d'un oligonucléotide choisi parmi les oligonucléotides ayant une séquence d'au moins 12 nucléotides naturels ou modifiés consécutifs incluse dans l'une des séquences SEQ ID NO1 à SEQ ID NO52, et parmi les oligonucléotides complémentaires de ces oligonucléotides, comme sonde pour la détection, dans un échantillon biologique, de bactéries appartenant à la famille des entérobactéries.

10. Utilisation selon la revendication 9, **caractérisée en ce que** la sonde comprend une séquence nucléotidique d'au moins 12 nucléotides naturels ou modifiés consécutifs incluse dans l'une des séquences SEQ ID NO1 à SEQ ID NO4, et leurs séquences complémentaires.

11. Utilisation selon la revendication 9, pour la détection, dans un échantillon biologique, d'au moins une espèce d'entérobactéries.

12. Utilisation selon la revendication 11, **caractérisée en ce que** la sonde comprend une séquence nucléotidique d'au moins 12 nucléotides naturels ou modifiés consécutifs incluse dans :
SEQ ID NO5 et contenant au moins 5 nucléotides naturels ou modifiés consécutifs inclus dans SEQ ID NO53,
SEQ ID NO6 et contenant au moins 5 nucléotides naturels ou modifiés consécutifs inclus dans SEQ ID NO54,
SEQ ID NO7 et contenant au moins 5 nucléotides naturels ou modifiés consécutifs inclus dans SEQ ID NO55,
SEQ ID NO8 et contenant au moins 5 nucléotides naturels ou modifiés consécutifs inclus dans SEQ ID NO56,
SEQ ID NO8 et contenant au moins 5 nucléotides naturels ou modifiés consécutifs inclus dans SEQ ID NO57,
SEQ ID NO9 et contenant au moins 5 nucléotides naturels ou modifiés consécutifs inclus dans SEQ ID NO58,
SEQ ID NO10 et contenant au moins 5 nucléotides naturels ou modifiés consécutifs inclus dans SEQ ID NO59,
SEQ ID NO11 et contenant au moins 5 nucléotides naturels ou modifiés consécutifs inclus dans SEQ ID NO60,
SEQ ID NO12 et contenant au moins 5 nucléotides naturels ou modifiés consécutifs inclus dans SEQ ID NO61,
SEQ ID NO13 et contenant au moins 5 nucléotides naturels ou modifiés consécutifs inclus dans SEQ ID NO62,
SEQ ID NO14 et contenant au moins 5 nucléotides naturels ou modifiés consécutifs inclus dans SEQ ID NO63,
SEQ ID NO15 et contenant au moins 5 nucléotides naturels ou modifiés consécutifs inclus dans SEQ ID NO64,
SEQ ID NO16 et contenant au moins 5 nucléotides naturels ou modifiés consécutifs inclus dans SEQ ID NO65,
SEQ ID NO17 et contenant au moins 5 nucléotides naturels ou modifiés consécutifs inclus dans SEQ ID NO64,
SEQ ID NO18 et contenant au moins 5 nucléotides naturels ou modifiés consécutifs inclus dans SEQ ID NO66,
SEQ ID NO19 et contenant au moins 5 nucléotides naturels ou modifiés consécutifs inclus dans SEQ ID NO67,
SEQ ID NO20 et contenant au moins 5 nucléotides naturels ou modifiés consécutifs inclus dans SEQ ID NO68,
SEQ ID NO21 et contenant au moins 5 nucléotides naturels ou modifiés consécutifs inclus dans SEQ ID NO69,
SEQ ID NO22 et contenant au moins 5 nucléotides naturels ou modifiés consécutifs inclus dans SEQ ID NO70,
SEQ ID NO23 et contenant au moins 5 nucléotides naturels ou modifiés consécutifs inclus dans SEQ ID NO71,
SEQ ID NO24 et contenant au moins 5 nucléotides naturels ou modifiés consécutifs inclus dans SEQ ID NO72,
SEQ ID NO25 et contenant au moins 5 nucléotides naturels ou modifiés consécutifs inclus dans SEQ ID NO73,
SEQ ID NO26 et contenant au moins 5 nucléotides naturels ou modifiés consécutifs inclus dans SEQ ID NO74,
SEQ ID NO27 et contenant au moins 5 nucléotides naturels ou modifiés consécutifs inclus dans SEQ ID NO75,
SEQ ID NO28 et contenant au moins 5 nucléotides naturels ou modifiés consécutifs inclus dans SEQ ID NO76,
ou une séquence complémentaire.

13. Utilisation selon la revendication 12, **caractérisée en ce que** la sonde comprend ou consiste en une séquence nucléotidique choisie parmi les séquences SEQ ID NO29 à SEQ ID NO52.

14. Utilisation selon l'une quelconque des revendications 9 à 13, **caractérisée par le fait que** la sonde est immobilisée sur un support solide.

15. Utilisation selon l'une quelconque des revendications 9 à 13, **caractérisée par le fait que** la sonde est marquée avec un agent traceur.

16. Procédé de détermination de la présence ou de l'absence d'au moins une entérobactérie, dans un échantillon contenant ou susceptible de contenir des acides nucléiques d'au moins une telle bactérie, **caractérisé en ce qu'**on met en contact ledit échantillon avec au moins une sonde telle que définie selon l'une des revendications 9 à 13 puis on détermine de façon connue en soi la formation ou l'absence de formation d'un complexe d'hybridation entre ladite sonde et l'acide nucléique de l'échantillon.

17. Procédé selon la revendication 16, pour la détermination de la présence ou l'absence d'une espèce ou d'un groupe d'espèces d'entérobactérie, **caractérisé en ce qu'**on utilise d'une part une première sonde telle que définie dans la revendication 10 et d'autre part une seconde sonde telle que définie dans la revendication 12 ou 13, étant entendu que lesdites première et seconde sondes sont capables de s'hybrider avec des régions non chevauchantes d'un acide nucléique correspondant au gène rpoB des entérobactéries.

18. Procédé selon la revendication 17, **caractérisé en ce que** ladite première sonde est telle que définie dans la revendication 14 et ladite deuxième sonde est telle que définie dans la revendication 15.

19. Procédé selon l'une quelconque des revendications 16 à 18, pour la détermination de la présence ou de l'absence de *Salmonella sofia,* **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO5 et contenant au moins 5 nucléotides naturels ou modifiés consécutifs inclus dans SEQ ID NO53,
SEQ ID NO27 et contenant au moins 5 nucléotides naturels ou modifiés consécutifs inclus dans SEQ ID NO75, et leurs séquences complémentaires.

20. Procédé selon la revendication 19, **caractérisé en ce que** la sonde a une séquence nucléotidique qui consiste ou qui comprend une séquence choisie parmi SEQ ID NO29, SEQ ID NO51 et, leurs séquences complémentaires.

21. Procédé selon l'une quelconque des revendications 16 à 18, pour la détermination de la présence ou de l'absence de *Salmonella typhimurium*, **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO6 et contenant au moins 5 nucléotides naturels ou modifiés consécutifs inclus dans SEQ ID NO54,
SEQ ID NO11 et contenant au moins 5 nucléotides naturels ou modifiés consécutifs inclus dans SEQ ID NO60,
SEQ ID NO24 et contenant au moins 5 nucléotides naturels ou modifiés consécutifs inclus dans SEQ ID NO72,
SEQ ID NO25 et contenant au moins 5 nucléotides naturels ou modifiés consécutifs inclus dans SEQ ID NO73, et leurs séquences complémentaires.

22. Procédé selon la revendication 21, **caractérisé en ce que** la sonde a une séquence nucléotidique qui consiste ou qui comprend une séquence choisie parmi SEQ ID NO30, SEQ ID NO35, SEQ ID NO48, SEQ ID NO49, SEQ ID NO52, et, leurs séquences complémentaires.

23. Procédé selon l'une quelconque des revendications 16 à 18, pour la détermination de la présence ou de l'absence de *Shigella dysenteriae* **caractérisé en ce qu'**on utilise une sonde choisie parmi celles comprenant une séquence nucléotidique d'au moins 12 nucléotides naturels ou modifiés consécutifs incluse dans SEQ ID NO7 et contenant au moins 5 nucléotides naturels ou modifiés consécutifs inclus dans SEQ ID NO55 et sa séquence complémentaire.

24. Procédé selon la revendication 23, **caractérisé en ce que** la sonde a une séquence nucléotidique qui consiste ou qui comprend une séquence choisie parmi SEQ ID NO31, et sa séquence complémentaire.

25. Procédé selon l'une quelconque des revendications 16 à 18, pour la détermination de la présence ou de l'absence de *Escherichia fergussoni*, **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO8 et contenant au moins 5 nucléotides naturels ou modifiés consécutifs inclus dans SEQ ID NO56,
SEQ ID NO8 et contenant au moins 5 nucléotides naturels ou modifiés consécutifs inclus dans SEQ ID NO57,
SEQ ID NO19 et contenant au moins 5 nucléotides naturels ou modifiés consécutifs inclus dans SEQ ID NO67, et leurs séquences complémentaires.

26. Procédé selon la revendication 25, **caractérisé en ce que** la sonde a une séquence nucléotidique qui consiste ou qui comprend une séquence choisie parmi SEQ ID NO32, SEQ ID NO43, et leurs séquences complémentaires.

27. Procédé selon l'une quelconque des revendications 16 à 18, pour la détermination de la présence ou de l'absence de *Enterobacter cloacae,* **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO9 et contenant au moins 5 nucléotides naturels ou modifiés consécutifs inclus dans SEQ ID NO58,
SEQ ID NO13 et contenant au moins 5 nucléotides naturels ou modifiés consécutifs inclus dans SEQ ID NO62,
SEQ ID NO16 et contenant au moins 5 nucléotides naturels ou modifiés consécutifs inclus dans SEQ ID NO65,
SEQ ID NO17 et contenant au moins 5 nucléotides naturels ou modifiés consécutifs inclus dans SEQ ID NO64,
SEQ ID NO18 et contenant au moins 5 nucléotides naturels ou modifiés consécutifs inclus dans SEQ ID NO66,
SEQ ID NO21 et contenant au moins 5 nucléotides naturels ou modifiés consécutifs inclus dans SEQ ID NO69,
SEQ ID NO22 et contenant au moins 5 nucléotides naturels ou modifiés consécutifs inclus dans SEQ ID NO70, et leurs séquences complémentaires.

28. Procédé selon la revendication. 27, **caractérisé en ce que** la sonde a une séquence nucléotidique qui consiste ou qui comprend une séquence choisie parmi SEQ ID NO33, SEQ ID NO37, SEQ ID NO40, SEQ ID NO41, SEQ ID NO42, SEQ ID NO45, SEQ ID NO46, et leurs sequences complémentaires.

29. Procédé selon l'une quelconque des revendications 16 à 18, pour la détermination de la présence ou de l'absence de *Klebsiella pneumoniae,* **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO10 et contenant au moins 5 nucléotides naturels ou modifiés consécutifs inclus dans SEQ ID NO59,
SEQ ID NO14 et contenant au moins 5 nucléotides naturels ou modifiés consécutifs inclus dans SEQ ID NO63,
SEQ ID NO15 et contenant au moins 5 nucléotides naturels ou modifiés consécutifs inclus dans SEQ ID NO64,
SEQ ID NO20 et contenant au moins 5 nucléotides naturels ou modifiés consécutifs inclus dans SEQ ID NO68,
SEQ ID NO23 et contenant au moins 5 nucléotides naturels ou modifiés consécutifs inclus dans SEQ ID NO71, et leurs séquences complémentaires.

30. Procédé selon la revendication 29, **caractérisé en ce que** la sonde a une séquence nucléotidique qui consiste ou qui comprend une séquence choisie parmi SEQ ID NO34, SEQ ID NO38, SEQ ID NO39, SEQ ID NO44, SEQ ID NO47, et leurs séquences complémentaires.

31. Procédé selon l'une quelconque des revendications 16 à 18, pour la détermination de la présence ou de l'absence de *Escherichia coli*, **caractérisé en ce qu'**on utilise une sonde choisie parmi celles comprenant une séquence nucléotidique d'au moins 12 nucléotides naturels ou modifiés consécutifs incluse dans l'une des séquences choisies parmi :
SEQ ID NO12 et contenant au moins 5 nucléotides naturels ou modifiés consécutifs inclus dans SEQ ID NO61,
SEQ ID NO26 et contenant au moins 5 nucléotides naturels ou modifiés consécutifs inclus dans SEQ ID NO74, et leurs séquences complémentaires.

32. Procédé selon la revendication 31, **caractérisé en ce que** la sonde a une séquence nucléotidique qui consiste ou qui comprend une séquence choisie parmi SEQ ID NO36, SEQ ID NO50, et leurs séquences complémentaires.

33. Procédé selon l'une quelconque des revendications 20 à 32 **caractérisé par le fait que** l'on utilise ladite sonde en combinaison avec une sonde telle que définie dans la revendication 10.

34. Amorce nucléotidique utilisable pour la synthèse d'un acide nucléique en présence d'une polymérase, **caractérisée en ce qu'**elle consiste en un oligonucléotide tel que défini à la revendication 1.

35. Sonde de thérapie, **caractérisée en ce qu'**elle consiste en un oligonucléotide tel que défini à la revendication 1.

## Patentansprüche

1. Oligonukleotid, ausgewählt aus den Oligonukleotiden, die eine Sequenz aus mindestens 12 natürlichen oder modifizierten aufeinander folgenden Nukleotiden umfassen, die in einer der Sequenzen SEQ ID Nr. 1 bis SEQ ID Nr. 52 enthalten ist und aus den ergänzenden Oligonukleotiden dieser Oligonukleotide, mit Ausnahme der Oligonukleotide, die eine Sequenz haben, die aus den folgenden Sequenzen ausgewählt wird: SEQ ID Nr. 28, SEQ ID Nr. 77, SEQ ID Nr. 78 und SEQ ID Nr. 79.

2. Oligonukleotid nach Anspruch 1, **dadurch gekennzeichnet, dass** es eine Nukleotidsequenz aus mindestens 12 natürlichen oder modifizierten aufeinander folgenden Nukleotiden umfasst, die in einer der Sequenzen SEQ ID NO1 bis SEQ ID NO4 enthalten ist.

3. Oligonukleotid nach Anspruch 1, **dadurch gekennzeichnet, dass** es eine Nukleotidsequenz aus mindestens 12 natürlichen oder modifizierten aufeinander folgenden Nukleotiden umfasst, die enthalten ist in:
SEQ ID NO5 und mindestens 5 natürliche oder modifizierte aufeinander folgende Nukleotide umfasst, die in SEQ ID NO53 enthalten sind,
SEQ ID NO6 und mindestens 5 natürliche oder modifizierte aufeinander folgende Nukleotide umfasst, die in SEQ ID NO54 enthalten sind,
SEQ ID NO8 und mindestens 5 natürliche oder modifizierte aufeinander folgende Nukleotide umfasst, die in SEQ ID NO56 enthalten sind,
SEQ ID NO8 und mindestens 5 natürliche oder modifizierte aufeinander folgende Nukleotide umfasst, die in SEQ ID NO57 enthalten sind,
SEQ ID NO9 und mindestens 5 natürliche oder modifizierte aufeinander folgende Nukleotide umfasst, die in SEQ ID NO58 enthalten sind,
SEQ ID NO10 und mindestens 5 natürliche oder modifizierte aufeinander folgende Nukleotide umfasst, die in SEQ ID NO59 enthalten sind,
SEQ ID NO12 und mindestens 5 natürliche oder modifizierte aufeinander folgende Nukleotide umfasst, die in SEQ ID NO61 enthalten sind,
SEQ ID NO13 und mindestens 5 natürliche oder modifizierte aufeinander folgende Nukleotide umfasst, die in SEQ ID NO62 enthalten sind,
SEQ ID NO14 und mindestens 5 natürliche oder modifizierte aufeinander folgende Nukleotide umfasst, die in SEQ ID NO63 enthalten sind,
SEQ ID NO16 und mindestens 5 natürliche oder modifizierte aufeinander folgende Nukleotide umfasst, die in SEQ ID NO65 enthalten sind,
SEQ ID NO17 und mindestens 5 natürliche oder modifizierte aufeinander folgende Nukleotide umfasst, die in SEQ ID NO64 enthalten sind,
SEQ ID NO18 und mindestens 5 natürliche oder modifizierte aufeinander folgende Nukleotide umfasst, die in SEQ ID NO66 enthalten sind,
SEQ ID NO19 und mindestens 5 natürliche oder modifizierte aufeinander folgende Nukleotide umfasst, die in SEQ ID NO67 enthalten sind,
SEQ ID NO20 und mindestens 5 natürliche oder modifizierte aufeinander folgende Nukleotide umfasst, die in SEQ ID NO68 enthalten sind,
SEQ ID NO21 und mindestens 5 natürliche oder modifizierte aufeinander folgende Nukleotide umfasst, die in SEQ ID NO69 enthalten sind,
SEQ ID NO22 und mindestens 5 natürliche oder modifizierte aufeinander folgende Nukleotide umfasst, die in SEQ ID NO70 enthalten sind,
SEQ ID NO23 und mindestens 5 natürliche oder modifizierte aufeinander folgende Nukleotide umfasst, die in SEQ ID NO71 enthalten sind,
SEQ ID NO24 und mindestens 5 natürliche oder modifizierte aufeinander folgende Nukleotide umfasst, die in SEQ ID NO72 enthalten sind,
SEQ ID NO25 und mindestens 5 natürliche oder modifizierte aufeinander folgende Nukleotide umfasst, die in SEQ ID NO73 enthalten sind,
SEQ ID NO26 und mindestens 5 natürliche oder modifizierte aufeinander folgende Nukleotide umfasst, die in SEQ ID NO74 enthalten sind,
SEQ ID NO27 und mindestens 5 natürliche oder modifizierte aufeinander folgende Nukleotide umfasst, die in SEQ ID NO75 enthalten sind.

4. Oligonukleotid nach Anspruch 3, **dadurch gekennzeichnet, dass** es eine Nukleotidsequenz umfasst oder aus ihr besteht, die aus den Sequenzen SEQ ID NO29 bis SEQ ID NO52 ausgewählt wird.

5. Sonde für das Erfassen von Bakterien in einer biologischen Probe, die zur Familie der Enterobakterien gehören, bestehend aus einem Oligonukleotid nach Anspruch 2.

6. Sonde bestehend aus einem Oligonukleotid nach Anspruch 3 oder 4 für das Erfassen mindestens einer Gattung Enterobakterien in einer biologischen Probe.

7. Sonde nach Anspruch 5 oder 6, **dadurch gekennzeichnet, dass** sie fest auf einem soliden Träger befestigt ist.

8. Sonde nach einem der Ansprüche 5 bis 7, **dadurch gekennzeichnet, dass** sie mit einem Indikatormittel markiert ist.

9. Einsatz eines Oligonukleotids, das aus Oligonukleotiden ausgewählt wird, die eine Sequenz aus mindestens 12 natürlichen oder modifizierten aufeinander folgenden Nukleotiden haben, die in einer der Sequenzen SEQ ID NO1 bis SEQ ID NO52 enthalten ist, und aus den ergänzenden Oligonukleotiden dieser Oligonukleotide, als Sonde zum Erfassen von Bakterien, die der Familie der Enterobakterien angehören, in einer biologischen Probe.

10. Einsatz nach Anspruch 9, **dadurch gekennzeichnet, dass** die Sonde eine Nukleotidsequenz aus mindestens 12 natürlichen oder modifizierten aufeinander folgenden Nukleotiden umfasst, die in einer der Sequenzen SEQ ID NO1 bis SEQ ID NO4 und ihrer ergänzenden Sequenzen enthalten ist.

11. Einsatz nach Anspruch 9, zum Erfassen mindestens einer Gattung Enterobakterien in einer biologischen Probe.

12. Einsatz nach Anspruch 11, **dadurch gekennzeichnet, dass** die Sonde eine Nukleotidsequenz aus mindestens 12 natürlichen oder modifizierten aufeinander folgenden Nukleotiden umfasst, die enthalten ist in:
SEQ ID NO5 und mindestens 5 natürliche oder modifizierte aufeinander folgende Nukleotide umfasst, die in SEQ ID NO53 enthalten sind,
SEQ ID NO6 und mindestens 5 natürliche oder modifizierte aufeinander folgende Nukleotide umfasst, die in SEQ ID NO54 enthalten sind,
SEQ ID NO7 und mindestens 5 natürliche oder modifizierte aufeinander folgende Nukleotide umfasst, die in SEQ ID NO55 enthalten sind,
SEQ ID NO8 und mindestens 5 natürliche oder modifizierte aufeinander folgende Nukleotide umfasst, die in SEQ ID NO56 enthalten sind,
SEQ ID NO8 und mindestens 5 natürliche oder modifizierte aufeinander folgende Nukleotide umfasst, die in SEQ ID NO57 enthalten sind,
SEQ ID NO9 und mindestens 5 natürliche oder modifizierte aufeinander folgende Nukleotide umfasst, die in SEQ ID NO58 enthalten sind,
SEQ ID NO10 und mindestens 5 natürliche oder modifizierte aufeinander folgende Nukleotide umfasst, die in SEQ ID NO59 enthalten sind,
SEQ ID N11 und mindestens 5 natürliche oder modifizierte aufeinander folgende Nukleotide umfasst, die in SEQ ID NO60 enthalten sind,
SEQ ID NO12 und mindestens 5 natürliche oder modifizierte aufeinander folgenden Nukleotide umfasst, die in SEQ ID NO61, enthalten sind,
SEQ ID NO13 und mindestens 5 natürliche oder modifizierte aufeinander folgende Nukleotide umfasst, die in SEQ ID NO62 enthalten sind,
SEQ ID NO14 und mindestens 5 natürliche oder modifizierte aufeinander folgende Nukleotide umfasst, die in SEQ ID NO63 enthalten sind,
SEQ ID NO15 und mindestens 5 natürliche oder modifizierte aufeinander folgende Nukleotide umfasst, die in SEQ ID NO64 enthalten sind,
SEQ ID NO16 und mindestens 5 natürliche oder modifizierte aufeinander folgende Nukleotide umfasst, die in SEQ ID NO65 enthalten sind,
SEQ ID NO17 und mindestens 5 natürliche oder modifizierte aufeinander folgende Nukleotide umfasst, die in SEQ ID NO64 enthalten sind,
SEQ ID NO18 und mindestens 5 natürliche oder modifizierte aufeinanderfolgende Nukleotide umfasst, die in SEQ ID NO66 enthalten sind,
SEQ ID NO19 und mindestens 5 natürliche oder modifizierte aufeinander folgende Nukleotide umfasst, die in SEQ ID NO67 enthalten sind,
SEQ ID NO20 und mindestens 5 natürliche oder modifizierte aufeinander folgende Nukleotide umfasst, die in SEQ ID NO68 enthalten sind,
SEQ ID NO21 und mindestens 5 natürliche oder modifizierte aufeinander folgende Nukleotide umfasst, die in SEQ ID NO69 enthalten sind,
SEQ ID NO22 und mindestens 5 natürliche oder modifizierte aufeinander folgende Nukleotide umfasst, die in SEQ ID NO70 enthalten sind,
SEQ ID NO23 und mindestens 5 natürliche oder modifizierte aufeinander folgende Nukleotide umfasst, die in SEQ ID NO71 enthalten sind,
SEQ ID NO24 und mindestens 5 natürliche oder modifizierte aufeinander folgende Nukleotide umfasst, die in SEQ ID NO72 enthalten sind,
SEQ ID NO25 und mindestens 5 natürliche oder modifizierte aufeinander folgende Nukleotide umfasst, die in SEQ ID NO73 enthalten sind,
SEQ ID NO26 und mindestens 5 natürliche oder modifizierte aufeinander folgende Nukleotide umfasst, die in SEQ ID NO74 enthalten sind,
SEQ ID NO27 und mindestens 5 natürliche oder modifizierte aufeinander folgende Nukleotide umfasst, die in SEQ ID NO75 enthalten sind,
SEQ ID NO28 und mindestens 5 natürliche oder modifizierte aufeinander folgende Nukleotide umfasst, die in SEQ ID NO76 oder einer ergänzenden Sequenz enthalten sind.

13. Einsatz nach Anspruch 12, **dadurch gekennzeichnet, dass** die Sonde eine Nukleotidsequenz umfasst oder aus ihr besteht, die aus den Sequenzen SEQ ID NO29 bis SEQ ID NO52 ausgewählt wird.

14. Einsatz nach einem der Ansprüche 9 bis 13, **dadurch gekennzeichnet, dass** die Sonde fest auf einem soliden Träger befestigt ist.

15. Einsatz nach einem der Ansprüche 9 bis 13, **dadurch gekennzeichnet, dass** die Sonde mit einem Indikatormittel markiert ist.

16. Verfahren zum Bestimmen der Anwesenheit oder des Fehlens mindestens einer Enterobakterie in einer Probe, die Nukleinsäuren mindestens einer solchen Bakterie enthält oder eventuell enthalten kann, **dadurch gekennzeichnet, dass** man die Probe mit mindestens einer Sonde nach einem der Ansprüche 9 bis 13 in Kontakt bringt und dann in an sich bekannter Weise das Bilden oder das Ausbleiben des Bildens eines Hybridisierungskomplexes zwischen der Sonde und der Nukleinsäure der Probe bestimmt.

17. Verfahren nach Anspruch 16 zum Bestimmen der Anwesenheit oder des Fehlens einer Gattung oder einer Gruppe Gattungen von Enterobakterien, **dadurch gekennzeichnet, dass** man einerseits eine erste Sonde verwendet, wie sie in Anspruch 10 definiert ist und, andererseits, eine zweite Sonde, wie sie in Anspruch 12 oder 13 definiert ist, wobei natürlich die erste und die zweite Sonde in der Lage sind, sich mit den nicht überlappenden Regionen einer Nukleinsäure zu hybridisieren, die dem Gen rpoB der Enterobakterien entspricht.

18. Verfahren nach Anspruch 17, **dadurch gekennzeichnet, dass** die erste Sonde wie in Anspruch 14 und die zweite Sonde wie in Anspruch 15 festgelegt beschaffen ist.

19. Verfahren nach einem der Ansprüche 16 bis 18, für die Bestimmung der Anwesenheit oder des Fehlens von *Salmonella sofia,* **dadurch gekennzeichnet, dass** man eine Sonde verwendet, die aus jenen ausgewählt wird, die eine Nukleotidsequenz aus mindestens 12 natürlichen oder modifizierten aufeinander folgenden Nukleotiden umfasst, die in einer der Sequenzen enthalten ist, die ausgewählt wird aus:
SEQ ID NO5 und mindestens 5 natürliche oder modifizierte aufeinander folgende Nukleotide umfasst, die in SEQ ID NO53 enthalten sind,
SEQ ID NO27 und mindestens 5 natürliche oder modifizierte aufeinander folgende Nukleotide umfasst, die in SEQ ID NO75 und ihren ergänzenden Sequenzen enthalten sind.

20. Verfahren nach Anspruch 19, **dadurch gekennzeichnet, dass** die Sonde eine Nukleotidsequenz hat, die aus einer Sequenz besteht oder diese umfasst, die aus SEQ ID NO29, SEQ ID NO51 und ihren ergänzenden Sequenzen ausgewählt wird.

21. Verfahren nach einem der Ansprüche 16 bis 18, zum Bestimmen der Anwesenheit oder des Fehlens von *Salmonella typhimurium,* **dadurch gekennzeichnet, dass** man eine Sonde ausgewählt aus jenen verwendet, die eine Nukleotidsequenz aus mindestens 12 natürlichen oder modifizierten aufeinander folgenden Nukleotiden umfassen, die in einer der Sequenzen enthalten ist, ausgewählt aus:
SEQ ID NO6 und die mindestens 5 natürliche oder modifizierte aufeinander folgende Nukleotide umfasst, die in SEQ ID NO54 enthalten sind,
SEQ ID NO11 und mindestens 5 natürliche oder modifizierte aufeinander folgende Nukleotide umfasst, die in SEQ ID NO60 enthalten sind,
SEQ ID NO24 und die mindestens 5 natürliche oder modifizierte aufeinander folgende Nukleotide umfasst, die in SEQ ID NO72 enthalten sind,
SEQ ID NO25 und die mindestens 5 natürliche oder modifizierte aufeinander folgende Nukleotide umfasst, die in SEQ ID NO73 und in ihren ergänzenden Sequenzen enthalten sind.

22. Verfahren nach Anspruch 21, **dadurch gekennzeichnet, dass** die Sonde eine Nukleotidsequenz hat, die aus einer Sequenz ausgewählt aus SEQ ID NO30, SEQ ID NO35, SEQ ID NO48, SEQ ID NO49, SEQ ID NO52 und ihren ergänzenden Sequenzen besteht oder diese umfasst.

23. Verfahren nach einem der Ansprüche 16 bis 18 zum Bestimmen der Anwesenheit oder des Fehlens von *Shigella dysenteriae*, **dadurch gekennzeichnet, dass** man eine Sonde, ausgewählt aus jenen verwendet, die eine Nukleotidsequenz von mindestens 12 natürlichen oder modifizierten aufeinander folgenden Nukleotiden umfassen und in SEQ ID NO7 enthalten ist, und die mindestens 5 natürliche oder modifizierte aufeinander folgende Nukleotide umfasst, die in SEQ ID NO55 und ihrer ergänzenden Sequenz enthalten sind.

24. Verfahren nach Anspruch 23, **dadurch gekennzeichnet, dass** die Sonde eine Nukleotidsequenz hat, die aus einer Sequenz ausgewählt aus SEQ ID NO31 und ihrer ergänzenden Sequenz besteht oder diese umfasst.

25. Verfahren nach einem der Ansprüche 16 bis 18, zum Bestimmen der Anwesenheit oder des Fehlens von Escherichia *fergussoni,* **dadurch gekennzeichnet, dass** man eine Sonde ausgewählt aus jenen verwendet, die eine Nukleotidsequenz aus mindestens 12 natürlichen oder modifizierten aufeinander folgenden Nukleotiden umfasst und in einer der Sequenzen enthalten ist, ausgewählt aus
SEQ ID NO8 und die mindestens 5 natürliche oder modifizierte aufeinander folgende Nukleotide umfasst, die in SEQ ID NO56 enthalten sind,
SEQ ID NO8 und die mindestens 5 natürliche oder modifizierte aufeinander folgende Nukleotide umfasst, die in SEQ ID NO57 enthalten sind,
SEQ ID NO19 und die mindestens 5 natürliche oder modifizierte aufeinander folgende Nukleotide umfasst, die in SEQ ID NO67 und ihren ergänzenden Sequenzen enthalten sind.

26. Verfahren nach Anspruch 25, **dadurch gekennzeichnet, dass** die Sonde eine Nukleotidsequenz hat, die aus einer Sequenz ausgewählt aus SEQ ID NO32, SEQ ID NO43 und ihren ergänzenden Sequenzen besteht oder diese umfasst.

27. Verfahren nach einem der Ansprüche 16 bis 18 zum Bestimmen der Anwesenheit oder des Fehlens von *Enterobacter cloacae,* **dadurch gekennzeichnet, dass** man eine Sonde ausgewählt aus jenen verwendet, die eine Nukleotidsequenz aus mindestens 12 natürlichen oder modifizierten aufeinander folgenden Nukleotiden umfasst, die in einer der Sequenzen enthalten sind, ausgewählt aus:
SEQ ID NO9 und die mindestens 5 natürliche oder modifizierte aufeinander folgende Nukleotide umfasst, die in SEQ ID NO58 enthalten sind,
SEQ ID NO13 und die mindestens 5 natürliche oder modifizierte aufeinander folgende Nukleotide umfasst, die in SEQ ID NO62 enthalten sind,
SEQ ID NO16 und die mindestens 5 natürliche oder modifizierte aufeinander folgende Nukleotide umfasst, die in SEQ ID NO65 enthalten sind,
SEQ ID NO17 und die mindestens 5 natürliche oder modifizierte aufeinander folgende Nukleotide umfasst, die in SEQ ID NO64 enthalten sind,
SEQ ID NO18 und die mindestens 5 natürliche oder modifizierte aufeinander folgende Nukleotide umfasst, die in SEQ ID NO66 enthalten sind,
SEQ ID NO21 und die mindestens 5 natürliche oder modifizierte aufeinander folgende Nukleotide umfasst, die in SEQ ID NO69 enthalten sind,
SEQ ID NO22 und die mindestens 5 natürliche oder modifizierte aufeinander folgende Nukleotide umfasst, die in SEQ ID NO70 und ihren ergänzenden Sequenzen enthalten sind.

28. Verfahren nach Anspruch 27, **dadurch gekennzeichnet, dass** die Sonde eine Nukleotidsequenz hat, die aus einer Sequenz ausgewählt aus SEQ ID NO33, SEQ ID NO37, SEQ ID NO40 SEQ ID NO41, SEQ ID NO42, SEQ ID NO45, SEQ ID NO46 und ihren ergänzenden Sequenzen besteht oder sie umfasst.

29. Verfahren nach einem der Ansprüche 16 bis 18 zum Bestimmen der Anwesenheit oder des Fehlens von *Klebsiella pneumoniae,* **dadurch gekennzeichnet, dass** man eine Sonde ausgewählt aus jenen verwendet, die eine Nukleotidsequenz aus mindestens 12 natürlichen oder modifizierten aufeinander folgenden Nukleotiden umfassen und in einer der Sequenzen enthalten sind, ausgewählt aus
SEQ ID NO10 und die mindestens 5 natürliche oder modifizierte aufeinander folgende Nukleotide umfasst, die in SEQ ID NO59 enthalten sind,
SEQ ID NO14 und die mindestens 5 natürliche oder modifizierte aufeinander folgende Nukleotide umfasst, die in SEQ ID NO63 enthalten sind,
SEQ ID NO15 und die mindestens 5 natürliche oder modifizierte aufeinander folgende Nukleotide umfasst, die in SEQ ID NO64 enthalten sind,
SEQ ID NO20 und die mindestens 5 natürliche oder modifizierte aufeinander folgende Nukleotide umfasst, die in SEQ ID NO68 enthalten sind,
SEQ ID NO23 und die mindestens 5 natürliche oder modifizierte aufeinander folgende Nukleotide umfasst, die in SEQ ID NO71 enthalten sind und ihren ergänzenden Sequenzen enthalten sind.

30. Verfahren nach Anspruch 29, **dadurch gekennzeichnet, dass** die Sonde eine Nukleotidsequenz hat, die aus einer Sequenz ausgewählt aus SEQ ID NO34, SEQ ID NO38, SEQ ID NO39, SEQ ID NO44, SEQ ID NO47 und ihren ergänzenden Sequenzen besteht oder diese umfasst.

31. Verfahren nach einem der Ansprüche 16 bis 18 zum Bestimmen der Anwesenheit oder des Fehlens von *Escherichia coli,* **dadurch gekennzeichnet, dass** man eine Sonde ausgewählt aus jenen verwendet, die eine Nukleotidsequenz aus mindestens 12 natürlichen oder modifizierten aufeinander folgenden Nukleotiden umfasst, die ihn einer der Sequenzen enthalten sind, ausgewählt aus:
SEQ ID NO12 und die mindestens 5 natürliche oder modifizierte aufeinander folgende Nukleotide umfasst, die in SEQ ID NO61 enthalten sind,
SEQ ID NO26 und die mindestens 5 natürliche oder modifizierte aufeinander folgende Nukleotide umfasst, die in SEQ ID NO74 enthalten sind und in ihren ergänzenden Sequenzen.

32. Verfahren nach Anspruch 31, **dadurch gekennzeichnet, dass** die Sonde eine Nukleotidsequenz hat, die aus einer Sequenz ausgewählt aus SEQ ID NO36, SEQ ID NO50 und ihren ergänzenden Sequenzen besteht oder diese umfasst.

33. Verfahren nach einem der Ansprüche 20 bis 32, **dadurch gekennzeichnet, dass** man die Sonde kombiniert mit einer Sonde verwendet, wie sie in Anspruch 10 definiert ist.

34. Nukleotidfragment verwendbar für die Synthese einer Nukleinsäure in Anwesenheit einer Polymerase, **dadurch gekennzeichnet, dass** sie aus einem Oligonukleotid wie in Anspruch 1 definiert besteht.

35. Therapiesonde, **dadurch gekennzeichnet, dass** sie aus einem Oligonukleotid wie in Anspruch 1 definiert besteht.

## Claims

1. Oligonucleotide chosen from the oligonucleotides comprising a sequence of at least 12 natural or modified consecutive nucleotides included in one of the sequences SEQ ID No. 1 to SEQ ID No. 52, and from the oligonucleotides complementary to these oligonucleotides, with the exclusion of the oligonucleotides having a sequence chosen from the following sequences: SEQ ID No. 28, SEQ ID No. 77, SEQ ID No. 78 and SEQ ID No. 79.

2. Oligonucleotide according to Claim 1, **characterized in that** it comprises a nucleotide sequence of at least 12 natural or modified consecutive nucleotides included in one of the sequences SEQ ID No. 1 to SEQ ID No. 4.

3. Oligonucleotide according to Claim 1, **characterized in that** it comprises a nucleotide sequence of at least 12 natural or modified consecutive nucleotides included in:
SEQ ID No. 5 and containing at least 5 natural or modified consecutive nucleotides included in SEQ ID No. 53,
SEQ ID No. 6 and containing at least 5 natural or modified consecutive nucleotides included in SEQ ID No. 54,
SEQ ID No. 8 and containing at least 5 natural or modified consecutive nucleotides included in SEQ ID No. 56,
SEQ ID No. 8 and containing at least 5 natural or modified consecutive nucleotides included in SEQ ID No. 57,
SEQ ID No. 9 and containing at least 5 natural or modified consecutive nucleotides included in SEQ ID No. 58,
SEQ ID No. 10 and containing at least 5 natural or modified consecutive nucleotides included in SEQ ID No. 59,
SEQ ID No. 12 and containing at least 5 natural or modified consecutive nucleotides included in SEQ ID No. 61,
SEQ ID No. 13 and containing at least 5 natural or modified consecutive nucleotides included in SEQ ID No. 62,
SEQ ID No. 14 and containing at least 5 natural or modified consecutive nucleotides included in SEQ ID No. 63,
SEQ ID No. 16 and containing at least 5 natural or modified consecutive nucleotides included in SEQ ID No. 65,
SEQ ID No. 17 and containing at least 5 natural or modified consecutive nucleotides included in SEQ ID No. 64,
SEQ ID No. 18 and containing at least 5 natural or modified consecutive nucleotides included in SEQ ID No. 66,
SEQ ID No. 19 and containing at least 5 natural or modified consecutive nucleotides included in SEQ ID No. 67,
SEQ ID No. 20 and containing at least 5 natural or modified consecutive nucleotides included in SEQ ID No. 68,
SEQ ID No. 21 and containing at least 5 natural or modified consecutive nucleotides included in SEQ ID No. 69,
SEQ ID No. 22 and containing at least 5 natural or modified consecutive nucleotides included in SEQ ID No. 70,
SEQ ID No. 23 and containing at least 5 natural or modified consecutive nucleotides included in SEQ ID No. 71,
SEQ ID No. 24 and containing at least 5 natural or modified consecutive nucleotides included in SEQ ID No. 72,
SEQ ID No. 25 and containing at least 5 natural or modified consecutive nucleotides included in SEQ ID No. 73,
SEQ ID No. 26 and containing at least 5 natural or modified consecutive nucleotides included in SEQ ID No. 74,
SEQ ID No. 27 and containing at least 5 natural or modified consecutive nucleotides included in SEQ ID No. 75.

4. Oligonucleotide according to Claim 3, **characterized in that** it comprises or consists of a nucleotide sequence chosen from the sequences SEQ ID No. 29 to SEQ ID No. 52.

5. Probe for detecting, in a biological sample, bacteria belonging to the family of enterobacteria, consisting of an oligonucleotide according to Claim 2.

6. Probe for detecting, in a biological sample, at least one species of enterobacteria, consisting of an oligonucleotide according to Claim 3 or 4.

7. Probe according to Claim 5 or 6, **characterized in that** it is immobilized on a solid support.

8. Probe according to any one of Claims 5 to 7, **characterized in that** it is labelled with a trace agent.

9. Use of an oligonucleotide chosen from the oligonucleotides having a sequence of at least 12 natural or modified consecutive nucleotides included in one of the sequences SEQ ID No. 1 to SEQ ID No. 52, and from the oligonucleotides complementary to these oligonucleotides, as a probe for detecting, in a biological sample, bacteria belonging to the family of enterobacteria.

10. Use according to Claim 9, **characterized in that** the probe comprises a nucleotide sequence of at least 12 natural or modified consecutive nucleotides included in one of sequences SEQ ID No. 1 to SEQ ID No. 4, and in sequences complementary thereto.

11. Use according to Claim 9, for detecting, in a biological sample, at least one species of enterobacteria.

12. Use according to Claim 11, **characterized in that** the probe comprises a nucleotide sequence of at least 12 natural or modified consecutive nucleotides included in:
SEQ ID No. 5 and containing at least 5 natural or modified consecutive nucleotides included in SEQ ID No. 53,
SEQ ID No. 6 and containing at least 5 natural or modified consecutive nucleotides included in SEQ ID No. 54,
SEQ ID No. 7 and containing at least 5 natural or modified consecutive nucleotides included in SEQ ID No. 55,
SEQ ID No. 8 and containing at least 5 natural or modified consecutive nucleotides included in SEQ ID No. 56,
SEQ ID No. 8 and containing at least 5 natural or modified consecutive nucleotides included in SEQ ID No. 57,
SEQ ID No. 9 and containing at least 5 natural or modified consecutive nucleotides included in SEQ ID No. 58,
SEQ ID No. 10 and containing at least 5 natural or modified consecutive nucleotides included in SEQ ID No. 59,
SEQ ID No. 11 and containing at least 5 natural or modified consecutive nucleotides included in SEQ ID No. 60,
SEQ ID No. 12 and containing at least 5 natural or modified consecutive nucleotides included in SEQ ID No. 61,
SEQ ID No. 13 and containing at least 5 natural or modified consecutive nucleotides included in SEQ ID No. 62,
SEQ ID No. 14 and containing at least 5 natural or modified consecutive nucleotides included in SEQ ID No. 63,
SEQ ID No. 15 and containing at least 5 natural or modified consecutive nucleotides included in SEQ ID No. 64,
SEQ ID No. 16 and containing at least 5 natural or modified consecutive nucleotides included in SEQ ID No. 65,
SEQ ID No. 17 and containing at least 5 natural or modified consecutive nucleotides included in SEQ ID No. 64,
SEQ ID No. 18 and containing at least 5 natural or modified consecutive nucleotides included in SEQ ID No. 66,
SEQ ID No. 19 and containing at least 5 natural or modified consecutive nucleotides included in SEQ ID No. 67,
SEQ ID No. 20 and containing at least 5 natural or modified consecutive nucleotides included in SEQ ID No. 68,
SEQ ID No. 21 and containing at least 5 natural or modified consecutive nucleotides included in SEQ ID No. 69,
SEQ ID No. 22 and containing at least 5 natural or modified consecutive nucleotides included in SEQ ID No. 70,
SEQ ID No. 23 and containing at least 5 natural or modified consecutive nucleotides included in SEQ ID No. 71,
SEQ ID No. 24 and containing at least 5 natural or modified consecutive nucleotides included in SEQ ID No. 72,
SEQ ID No. 25 and containing at least 5 natural or modified consecutive nucleotides included in SEQ ID No. 73,
SEQ ID No. 26 and containing at least 5 natural or modified consecutive nucleotides included in SEQ ID No. 74,
SEQ ID No. 27 and containing at least 5 natural or modified consecutive nucleotides included in SEQ ID No. 75,
SEQ ID No. 28 and containing at least 5 natural or modified consecutive nucleotides included in SEQ ID No. 76,
or a complementary sequence.

13. Use according to Claim 12, **characterized in that** the probe comprises or consists of a nucleotide sequence chosen from the sequences SEQ ID No. 29 to SEQ ID No. 52.

14. Use according to any one of Claims 9 to 13, **characterized in that** the probe is immobilized on a solid support.

15. Use according to any one of Claims 9 to 13, **characterized in that** the probe is labelled with a trace agent.

16. Method for determining the presence or absence of at least one enterobacterium, in a sample containing or liable to contain nucleic acids of at least one such bacterium, **characterized in that** said sample is brought into contact with at least one probe as defined in any one of Claims 9 to 13, and then the formation or the lack of formation of a hybridization complex between said probe and the nucleic acid of the sample is determined in a manner known per se.

17. Method according to Claim 16, for determining the presence or absence of a species or of a group of species of enterobacterium **characterized in that** use is made, firstly, of a first probe as defined in Claim 10 and, secondly, a second probe as defined in Claim 12 or 13, it being understood that said first and second probes are capable of hybridizing with non-overlapping regions of a nucleic acid corresponding to the rpoB gene of enterobacteria.

18. Method according to Claim 17, **characterized in that** said first probe is as defined in Claim 14 and said second probe is as defined in Claim 15.

19. Method according to any one of Claims 16 to 18, for determining the presence or absence of *Salmonella sofia,* **characterized in that** use is made of a probe chosen from those comprising a nucleotide sequence of at least 12 natural or modified consecutive nucleotides included in one of the sequences chosen from:
SEQ ID No. 5 and containing at least 5 natural or modified consecutive nucleotides included in SEQ ID No. 53,
SEQ ID No. 27 and containing at least 5 natural or modified consecutive nucleotides included in SEQ ID No. 75,
and the sequences complementary thereto.

20. Method according to Claim 19, **characterized in that** the probe has a nucleotide sequence which consists of or comprises a sequence chosen from SEQ ID No. 29 and SEQ ID No. 51, and the sequences complementary thereto.

21. Method according to any one of Claims 16 to 18, for determining the presence or absence of *Salmonella typhimurium*, **characterized in that** use is made of a probe chosen from those comprising a nucleotide sequence of at least 12 natural or modified consecutive nucleotides included in one of the sequences chosen from:
SEQ ID No. 6 and containing at least 5 natural or modified consecutive nucleotides included in SEQ ID No. 54,
SEQ ID No. 11 and containing at least 5 natural or modified consecutive nucleotides included in SEQ ID No. 60,
SEQ ID No. 24 and containing at least 5 natural or modified consecutive nucleotides included in SEQ ID No. 72,
SEQ ID No. 25 and containing at least 5 natural or modified consecutive nucleotides included in SEQ ID No. 73,
and the sequences complementary thereto.

22. Method according to Claim 21, **characterized in that** the probe has a nucleotide sequence which consists of or which comprises a sequence chosen from SEQ ID No. 30, SEQ ID No. 35, SEQ ID No. 48, SEQ ID No. 49 and SEQ ID No. 52, and the sequences complementary thereto.

23. Method according to any one of Claims 16 to 18, for determining the presence or absence of *Shigella dysenteriae,* **characterized in that** use is made of a probe chosen from those comprising a nucleotide sequence with at least 12 natural or modified consecutive nucleotides included in SEQ ID No. 7 and containing at least 5 natural or modified consecutive nucleotides included in SEQ ID No. 55, and in sequence complementary thereto.

24. Method according to Claim 23, **characterized in that** the probe has a nucleotide sequence which consists of or which comprises a sequence chosen from SEQ ID No. 31 and a sequence complementary thereto.

25. Method according to any one of Claims 16 to 18, for determining the presence or absence of *Escherichia fergussoni,* **characterized in that** use is made of a probe chosen from those comprising a nucleotide sequence of at least 12 natural or modified consecutive nucleotides included in one of the sequences chosen from:
SEQ ID No. 8 and containing at least 5 natural or modified consecutive nucleotides included in SEQ ID No. 56,
SEQ ID No. 8 and containing at least 5 natural or modified consecutive nucleotides included in SEQ ID No. 57,
SEQ ID No. 19 and containing at least 5 natural or modified consecutive nucleotides included in SEQ ID No. 67,
and the sequences complementary thereto.

26. Method according to Claim 25, **characterized in that** the probe has a nucleotide sequence which consists of or which comprises a sequence chosen from SEQ ID No. 32 and SEQ ID No. 43, and the sequences complementary thereto.

27. Method according to any one of Claims 16 to 18, for determining the presence or absence of *Entero* *bacter cloacae,* **characterized in that** use is made of a probe chosen from those comprising a nucleotide sequence of at least 12 natural or modified consecutive nucleotides included in one of the sequences chosen from:
SEQ ID No. 9 and containing at least 5 natural or modified consecutive nucleotides included in SEQ ID No. 58,
SEQ ID No. 13 and containing at least 5 natural or modified consecutive nucleotides included in SEQ ID No. 62,
SEQ ID No. 16 and containing at least 5 natural or modified consecutive nucleotides included in SEQ ID No. 65,
SEQ ID No. 17 and containing at least 5 natural or modified consecutive nucleotides included in SEQ ID No. 64,
SEQ ID No. 18 and containing at least 5 natural or modified consecutive nucleotides included in SEQ ID No. 66,
SEQ ID No. 21 and containing at least 5 natural or modified consecutive nucleotides included in SEQ ID No. 69,
SEQ ID No. 22 and containing at least 5 natural or modified consecutive nucleotides included in SEQ ID No. 70,
and the sequences complementary thereto.

28. Method according to Claim 27, **characterized in that** the probe has a nucleotide sequence which consists of or which comprises a sequence chosen from SEQ ID No. 33, SEQ ID No. 37, SEQ ID No. 40, SEQ ID No. 41, SEQ ID No. 42, SEQ ID No. 45 and SEQ ID No. 46, and the sequences complementary thereto.

29. Method according to any one of Claims 16 to 18, for determining the presence or absence of *Klebsiella pneumoniae,* **characterized in that** use is made of a probe chosen from those comprising a nucleotide sequence of at least 12 natural or modified consecutive nucleotides included in one of the sequences chosen from:
SEQ ID No. 10 and containing at least 5 natural or modified consecutive nucleotides included in SEQ ID No. 59,
SEQ ID No. 14 and containing at least 5 natural or modified consecutive nucleotides included in SEQ ID No. 63,
SEQ ID No. 15 and containing at least 5 natural or modified consecutive nucleotides included in SEQ ID No. 64,
SEQ ID No. 20 and containing at least 5 natural or modified consecutive nucleotides included in SEQ ID No. 68,
SEQ ID No. 23 and containing at least 5 natural or modified consecutive nucleotides included in SEQ ID No. 71,
and the sequences complementary thereto.

30. Method according to Claim 29, **characterized in that** the probe has a nucleotide sequence which consists of or which comprises a sequence chosen from SEQ ID No. 34, SEQ ID No. 38, SEQ ID No. 39, SEQ ID No. 44 and SEQ ID No. 47, and the sequences complementary thereto.

31. Method according to any one of Claims 16 to 18, for determining the presence or absence of *Escherichia coli,* **characterized in that** use is made of a probe chosen from those comprising a nucleotide sequence of at least 12 natural or modified consecutive nucleotides included in one of the sequences chosen from:
SEQ ID No. 12 and containing at least 5 natural or modified consecutive nucleotides included in SEQ ID No. 61,
SEQ ID No. 26 and containing at least 5 natural or modified consecutive nucleotides included in SEQ ID No. 74,
and the sequences complementary thereto.

32. Method according to Claim 31, **characterized in that** the probe has a nucleotide sequence which consists of or which comprises a sequence chosen from SEQ ID No. 36 and SEQ ID No. 50, and the sequences complementary thereto.

33. Method according to any one of Claims 20 to 32, **characterized in that** use is made of said probe in combination with a probe as defined in Claim 10.

34. Nucleotide primer which can be used for synthesizing a nucleic acid in the presence of a polymerase, **characterized in that** it consists of an oligonucleotide as defined in Claim 1.

35. Probe for therapy, **characterized in that** it consists of an oligonucleotide as defined in Claim 1.
